# EUROPÄISCHE PATENTSCHRIFT

(11) **EP 2 450 025 B1**
(45) Veröffentlichungstag und Bekanntmachung des Hinweises auf die Patenterteilung: **28.11.2012**
(21) Anmeldenummer: 11188086.0
(22) Anmeldetag: 07.11.2011
(51) Int. Cl.: A61K 6/00, C07F 9/06, C09D 133/00, C07F 9/09, C07F 9/24

(54) **Polymerisierbare Phosphorsäurederivate umfassend ein polyalicyclisches Strukturelement**
Polymerisable phosphoric acid derivatives comprising a polyalicyclic structure element
Dérivés d'acide phosphorique polymérisables comprenant un élément structurel polyalicyclique

(30) Priorität: 08.11.2010 DE 102010043571
(43) Veröffentlichungstag der Anmeldung: 09.05.2012
(73) Patentinhaber: VOCO GmbH, 27472 Cuxhaven (DE)
(72) Erfinder: Stepputtis, Dr. Manfred, 27449 Kutenholz (DE); Blömker, Dr. Tobias, 27472 Cuxhaven (DE); Plaumann, Manfred Thomas, 27476 Cuxhaven (DE)
(74) Vertreter: Eisenführ, Speiser & Partner

(56) Entgegenhaltungen:
- EP-A1- 1 454 911
- WO-A1-2006/111373
- JP-A- 2007 091 642

## Beschreibung

Die vorliegende Erfindung betrifft bestimmte neue polymerisierbare Phosphorsäurederivate (nachfolgend auch als Monomere bezeichnet) umfassend ein polyalicyclisches Strukturelement, Mischungen umfassend eine oder mehrere dieser Verbindungen und entsprechende härtbare Gemische und Erzeugnisse sowie deren jeweilige Verwendung als Dentalmaterial bzw. zur Herstellung eines Dentalmaterials. Die Verbindungen eignen sich hervorragend als Haftvermittler, insbesondere in dentalen Adhäsivmaterialien. Die Erfindung betrifft ferner ein Verfahren zur Herstellung dieser Verbindungen bzw. Mischungen und ein Verfahren zur Herstellung eines Erzeugnisses, vorzugsweise eines für den Dentalbereich geeigneten Erzeugnisses.

Über einen weiten Zeitraum hinweg wurde die zahnärztliche Füllungstherapie durch den Einsatz von Amalgam bestimmt. Da Amalgam keine eigene Haftung an Zahnhartsubstanz aufweist, muss der Füllungswerkstoff mechanisch, beispielsweise durch die Präparation von Unterschnitten oder Verkeilungen bei der definitiven Versorgung von Kavitäten zum Halten und Verbleib in der Kavität gebracht werden. Ein solches Verfahren verbraucht jedoch gesunde Zahnhartsubstanz im Übermaß und wird dem heutigen Standard der restaurativen Zahnheilkunde mit der Forderung nach minimalinvasiven Präparationen und möglichst substanzschonenden Kavitätendesign nicht mehr gerecht. Weil Amalgam keine Eigenhaftung zur Zahnhartsubstanz zeigt, tritt beim Einsatz von Amalgam stets ein Randspalt zwischen der Präparation und dem Zahnfüllmaterial auf. Dieser Randspalt wird in den der Füllungslegung folgenden Wochen durch bakterizide Korrosionsprodukte des Amalgams und sonstigen Ablagerungen weitgehend aufgefüllt und ein Eindringen von kariogenen Keimen wirksam verhindert. Je größer der Randspalt ausfällt, desto geringer ist der Anteil an Amalgamkorrosionsprodukten im Spalt und ein Eindringen von Bakterien wird immer wahrscheinlicher. Ein breiter Randspalt führt zu einer raschen Besiedlung mit Mikroorganismen, die zur Ausbildung einer Sekundärkaries und damit zum Füllungsverlust führen können. Zusätzlich gilt das Amalgam toxikologisch als bedenklich und ist zudem vom ästhetischen Gesichtspunkt aus völlig unbefriedigend.

Dies sind wichtige Gründe, warum ein stetig wachsender Anteil der Patienten zahnfarbene Kunststoffmaterialien oder andere Alternativen dem Amalgam zur Versorgung ihrer Kavitäten vorzieht.

Mit dem Aufkommen von härtbaren, plastischen Kunststoffen als alternative Füllungsmaterialien in der konservierenden Zahnheilkunde ergaben sich neue verschiedene Problemfelder, die bis heute noch nicht umfassend zufrieden stellend gelöst sind. Zu den Schwachpunkten plastischer Füllungskunststoffe zählen außer dem erhöhten Abrieb und der biologischen Verträglichkeit der so genannte Polymerisationsschrumpf und das Problem des dauerhaften, randspaltfreien Verbundes mit der Zahnhartsubstanz, welches die Haltbarkeit der Restauration häufig gefährdet.

Werden Kunststoffe ausgehärtet, so findet beim Übergang von der flüssigen zu der festen Phase eine Änderung der Dichte statt. Dieses Phänomen, auch Schrumpf genannt, kann dazu führen, dass eine dauerhafte und feste Verbindung zwischen der Zahnsubstanz und dem Füllungsmaterial nicht aufgebaut werden kann, da durch die Polymerisationsschrumpfung hohe Zugkräfte auf den Kunststoff-Zahnsubstanz-Verbund wirken. Dieser Effekt kann außerdem durch zeitlich versetzte, gegenläufige Quellungseffekte durch Aufnahme oraler Flüssigkeiten weiter kompliziert werden. Die Aufnahme von Flüssigkeiten aus dem Mundraum wird in erster Linie durch die Polarität der Kunststoffkomponenten der Füllung und des Adhäsivs bestimmt. Hoch polare Materialien neigen zur Aufnahme größerer Mengen Wasser aus dem Speichel und führen zu einer Volumenzunahme sowie zur allmählichen Ablösung des Kunststoffes von der Zahnhartsubstanz. Die aufgenommene Feuchtigkeit kann darüber hinaus Hydrolyseprozesse in Gang setzen, die häufig organische oder anorganisch-organische Ester betreffen und zu einer beträchtlichen Schwächung des Haftverbundes und der physikalischen Eigenschaften der Füllung führen können, die sich dann nachteilig auf die Langzeitprognosen der Haltbarkeit auswirken. Besitzt das Füllungsmaterial keine ausreichende Haftkraft am Kavitätenrand, so bilden sich feine Randspalte zwischen dem Zahn und der Restauration, die häufig für Hypersensibilitäten verantwortlich sind und ein Eindringen von Flüssigkeiten und Bakterien an der Dentin-Kunstoffgrenze entlang der Kavitätenwand in die Tiefe ermöglichen. Eine solche unzureichende Randadaption des dentalen Füllungskomposits kann demnach eine bakterielle Unterminierung der Restauration mit nachfolgender Sekundärkariesbildung und schwerer Schädigung des Zahnes verursachen. Die Folgen können von einer Randverfärbung über eine pulpitische Reizung bis hin zur Zahnmark- und Zahnwurzelvereiterung reichen, die letztendlich zum Verlust der Restauration und gegebenenfalls des Zahnes führen.

Fortschritte im Verfahren der adhäsiven Bindung von Füllungsmaterialien an das harte Zahngewebe erfolgten in der Vergangenheit nur in kleinen Schritten und sind in der Literatur beschrieben.

Ein Grund für den unzureichenden Verbund zwischen Zahnsubstanz und Kunststoff liegt in der Struktur des Dentins begründet, die als Folge eines zur Mundhöhle gerichteten osmotischen Drucks des Dentinliquors in den Dentintubuli immer eine gewisse Feuchtigkeit aufweist. Darüber hinaus besteht das Dentin zu einem großen Teil aus organischen Substanzen, insbesondere Kollagen, in dem die anorganischen Hydroxylapatitkristalle eingelagert sind. Dieser Strukturtyp ist sehr viel komplizierter aufgebaut als der Zahnschmelz. Zudem entsteht bei der Präparation am Dentin auf der Oberfläche eine Schmierschicht, die aus Zahnhartsubstanzbestandteilen, Bakterien, Speichel und Blut besteht und weder mechanisch noch durch Spülung zu entfernen ist.

Diese Voraussetzungen erschweren den Aufbau eines beständigen Verbundes zwischen Zahnsubstanz und Kunststoff in ganz erheblichem Maße.

Eine erste Überlegung führte zur Verwendung oberflächenaktiver Monomere als Verbindungsmittel zwischen der hydrophilen Zahnsubstanz und dem hydrophoben Füllungskunststoff. US 3,200,142 schlug das Additionsprodukt zwischen der Aminosäure N-Phenylglycin und Glycidylmethacrylat (NPG-GMA) als Mittel zur Verbesserung der adhäsiven Bindung vor. Die eine endständige Carbonsäurefunktion von N-(2-hydroxy-3-methacryloxypropyl)-N-phenylglycin soll als funktionelle Gruppe eine Bindung zu den Calciumionen des im anorganischen Bestandteil des Dentins enthaltenen Hydroxylapatits aufbauen, während die ethylenische Doppelbindung der Methacrylatgruppe eine kovalente Bindung zum Füllungskunststoff während der Polymerisation sicherstellen soll. Besonders wirksam soll das Addukt in Form seiner Carbonsäuresalze sein.

In ähnlichen Ansätzen wurden monomere Bindungsagentien eingesetzt, die außer der Carbonsäurefunktion (oder einer zu einer Cabonsäurefunktion überführbaren Gruppe) auch andere oberflächenaktive Struktureinheiten wie Phosphat-, Sulfonat-, Sulfinat-, Isocyanat-, Hydroxyl- und Amidgruppen enthalten konnten.

Ein alternativer Ansatz zum Aufbau eines randspaltfreien Verbundes zwischen Füllungskunststoff und der Zahnhartsubstanz bestand darin, Haftsysteme zu formulieren, die sich mit den organischen Bestandteilen des Dentins, dem Kollagen, und einer endständigen Gruppe des Haftvermittlers umsetzen konnten. Eingesetzt wurden hier beispielsweise Aldehydgruppen aufweisende Verbindungen wie Glutaraldehyd (EP 0 141 324). Die Aldehydfunktion soll beispielsweise mit einer Aminofunktion des Proteins im Kollagen in einem ersten Schritt zu einem Aminoalkohol umgesetzt werden, um dann im zweiten Schritt unter Abspaltung von Wasser zu einer Schiffschen Base abzureagieren. Es wurde weiterhin vorgeschlagen, die aldehydgruppenhaltigen Verbindungen zusammen mit aktiven Wasserstoffatomen ausgestatteten Monomeren, wie beispielsweise Hydroxyethylmethacrylat, einzusetzen, um sicherzustellen, dass die Eliminierung von Wasser und somit die Umsetzung der zweiten Reaktionsstufe und Kopplung des Haftmonomers an das Dentin tatsächlich stattfindet, weil Schiffsche Basen unter den wässrigen Bedingungen der Mundhöhle möglicherweise nicht ausreichend stabil sind.

Ferner wurde versucht, zusätzlich zur Aldehydreaktion eine gezielte Pfropfung des Kollagens durch mit Tri-n-butylboran initiierte Pfropfcopolymerisation von Methacrylsäureestern durchzuführen (US 4,830,616).

Eine weitere Verfeinerung des Systems beschreibt die DE 41 37 076. Anstelle Aldehydgruppen-aufweisender Verbindungen wurden hier β-Dicarbonylverbindungen, wie beispielsweise 2-Acetoacetoxyethylmethacrylat, eingesetzt. Es wurde angenommen, dass die β-Carbonylfunktion eine erheblich größere Reaktivität gegenüber dem Protein, d.h. dem Kollagen, im Vergleich zur Aldehydgruppe aufweist und zusätzlich die guten Komplexierungseigenschaften der β-Carbonylgruppe eine Rolle spielen.

Weiterhin wurde versucht, durch eine Kombination der Strategien Adhäsivzusammensetzungen bereitzustellen, die sowohl an Kollagen als auch an Calcium haften (EP 0 321 683).

In der Praxis zeigte sich jedoch, dass die Haftwerte der oben genannten Systeme nach kurzer Zeit stark nachließen. Im weiteren Verlauf des Bemühens, geeignete und zuverlässige Haftmonomere zu entwickeln, konnten schließlich einige wenige und ganz spezielle Verbindungen aufgefunden werden, die eine erhöhte Haftwirkung zwischen der Zahnsubstanz und dem Füllungsmaterial auch nach Alterung der Systeme aufwiesen. Diese Verbindungen wurden dann auch in Dentalmaterialien eingesetzt und kommerziell vertrieben. Diese aktuellen Haftmittel erfordern verschiedene Verarbeitungsmethoden, um erfolgreich mit Zahnhartsubstanzen verbunden zu werden. Allen gemeinsam ist die Veränderung des Schmelzes oder Dentins durch Säuren, Primer oder Conditioner, deren Aufgabe es, vereinfacht gesprochen, ist, die Oberfläche durch Schaffung retentiver Muster aufzurauhen. Diese Ätzung erfolgt zumeist in einem separaten Schritt. Flüssige unpolare Harzmischungen können dann, häufig vermittelt durch polare, flüchtige Lösungsmittel, in die retentiven Oberflächen einziehen und gehärtet werden. Die verwendeten Säuren, Primer und Conditioner enthalten häufig organische oder anorganische Säuren wie beispielsweise Phosphorsäure oder Zitronensäure, die aufgrund ihres niedrigen pH-Wertes die anorganischen Bestandteile über einen bestimmten Zeitraum auflösen und dann entfernt werden müssen. In anderen modernen Bond-Materialien wird die Ätzung des Haftgrundes mit dem Auftragen des Adhäsivs durch die Verwendung haftungsfördernder, saurer und polymerisierbarer Verbindungen kombiniert.

Als eine Haftung vermittelnde Verbindung aus der Gruppe der Mono- oder Diphosphatester der Hydroxyalkylmethacrylate erwies sich 10-(Meth)acryloyloxydecyldihydrogenphosphat (10-MDP) (EP 0 074 708, EP 1 084 131). Die Phosphorsäurefunktion bildet mit dem Hydroxylapatit stabile, wasserunlösliche Salze, wobei das Calcium mit Hilfe der Phosphorsäuregruppe komplexiert wird. Der Methylenspacer scheint eine genau abgestimmte Länge aufzuweisen, um gegenseitige Störungen durch sterische Effekte bei der Bindungsbildung an beiden Enden des Haftvermittlers zu vermeiden. Dies wiederum scheint eine Voraussetzung zu sein, um die Substratoberfläche optimal und gleichmäßig benetzen zu können.

Präparativ kann 10-MDP durch Reaktion von 10-Hydroxydecyl(meth)acrylat mit Phosphoroxychlorid erhalten werden.

Unter (Meth)acryl ist im Rahmen des vorliegenden Textes sowohl Acryl als auch Methacryl zu verstehen.

Weitere Verbindungen dieses Typs sind beispielsweise 2-(Meth)acryloyloxyethyldihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogenphosphat, 2-(Meth)acryloyloxynonyldihydrogenphosphat, 11-(Meth)acryloyloxyundecyldihydrogenphosphat, 20-(Meth)acryloyloxyeicosyldihydrogenphosphat, 1,3-Di(meth)acyloyloxypropyl-2-dihydrogenphosphat oder 2-(Meth)acryloyloxyethylphenyldihydrogenphosphat.

Anstelle eines Phosphorsäurerestes können die polymerisierbaren Monomeren auch einen Diphosphorsäurerest aufweisen, wie beispielsweise das Di(2-(meth)acryloyloxyethyl)pyrophosphat, das Di(2-(meth)acryloyloxypropyl)pyrophosphat, das Di(2-(meth)acryloyloxybutyl)pyrophosphat, das Di(2-(meth)acryloyloxypentyl)pyrophosphat, das Di(2-(meth)acryloyloxyhexyl)pyrophosphat, das Di(2-(meth)acryloyloxydecyl)pyrophosphat, etc.. Es können auch die entsprechenden Säurehalogenide eingesetzt werden.

Neben den polymerisierbaren Monomeren mit einem Phosphorsäure- oder Pyrophosphorsäurerest können entsprechende Verbindungen eingesetzt werden, die einen Phosphonsäure-, einen Thiophosphorsäure- oder einen Sulfonsäurerest aufweisen.

Analog können Haftung vermittelnde Monomere beispielsweise aus Hydroxyalkylmethacrylat oder Glyceryldimethacrylat synthetisiert werden. So bilden sich beispielsweise bei den Umsetzungen von Hydroxyethylmethacrylat mit Phosphoroxychlorid Gemische, die Mono-, Di- und Triester aufweisen.

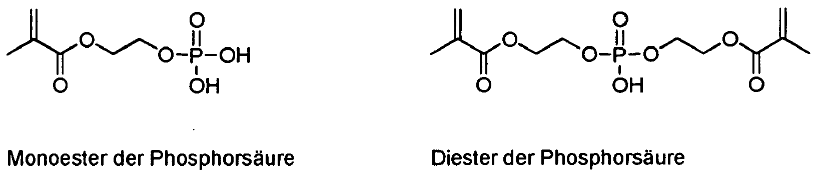

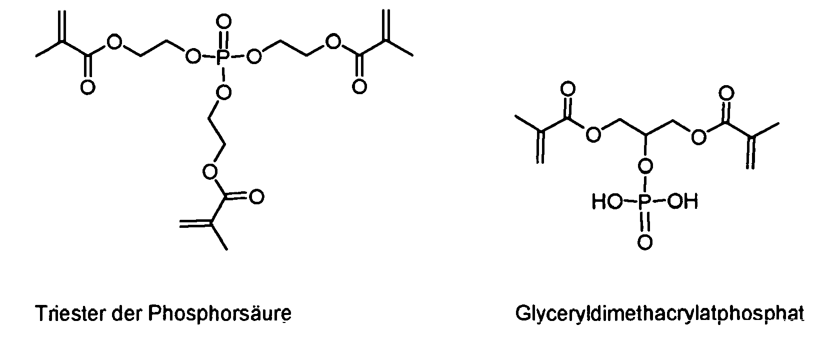

Ein weiterer Typ eines Haftmonomers ist der Phosphorsäureester von Pentaerythritoltriacrylat oder von Dipentaerythritolpentaacrylat (PENTA, US 4,514,342). Der Ester wird aus Dipentaerythritolmonohydroxypentaacrylat und Phosphoroxychlorid in Gegenwart von Triethylamin hergestellt.

Andere, Haftung vermittelnde Verbindungen sind Methacryloyloxyalkylderivate aromatischer Carbonsäuren. Es zeigte sich, dass insbesondere Trimellitsäure-4-methacryloyloxyethylester (4-MET) oder Trimellitsäureanhydrid-4-methacryloyloxyethylester (4-META) als haftungsfördernde Monomere eingesetzt werden können (DE 28 28 381, US 4,148,988, EP 0 684 033, EP 0 684 034). 4-META kann durch Dehydrochlorierungsreaktion zwischen Hydroxyethylmethacrylat und wasserfreiem Trimellitsäurechlorid oder durch Dehydrationsreaktion zwischen 2-Hydroxyethylmethacrylat und Trimellitsäureanhydrid hergestellt werden.

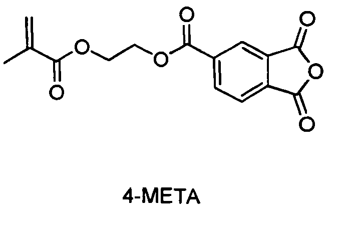

Ananlog sollen Pyromellitsäuredimethacrylat sowie Pyromellitsäureglyceroldimethacrylat ebenfalls als Haftmonomer geeignet sein.

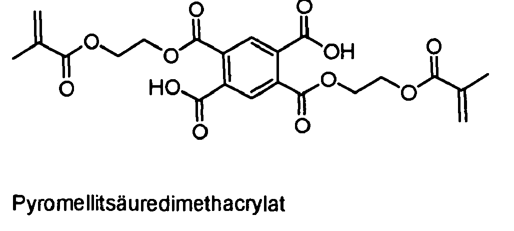

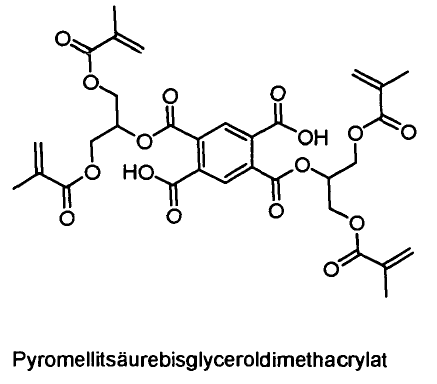

Andere Methacryloyloxyethylderivate aromatischer Carbonsäuren, die als Haftmonomere geeignet sein sollen, sind entsprechende Verbindungen der Phthalsäure.

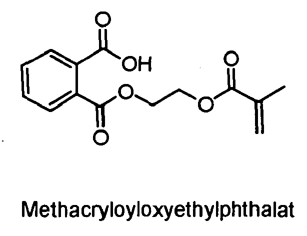

Des Weiteren sollen Methacryloyloxyethylderivate der Bernsteinsäure und der Maleinsäure als Haftmonomer einsetzbar sein.

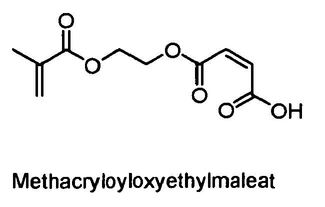

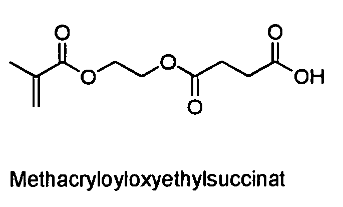

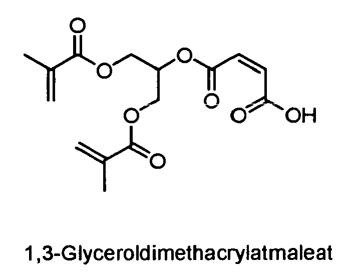

Weitere reaktive Haftkomponenten werden in EP 1 148 060, EP 0 909 761 und EP 1 148 071 offenbart, wo polymerisierbare und hydrolysestabile Acrylphosphonsäuren beschrieben sind. Der aufwendige Syntheseweg beginnt mit der Umsetzung von Formaldehyd und einem geeigneten Acrylsäureester in Gegenwart eines Katalysators unter Bildung einer Methylolgruppe in α-Position des Esters und anschließender Halogenierung des Alkohols mit einem anorganischen Säurehalogenid. Der so hergestellte α-Halogenmethacrylsäureester wird dann mit geeigneten und zuvor geschützten mono-oder difunktionellen Phosphonsäureestern umgesetzt. Nach Abspaltung der Schutzgruppe wird dann die polymerisierbare und hydrolysestabile Acrylphosphonsäure erhalten, deren Merkmal die endständige Oxo-ethylacrylatfunktion darstellt.

In der EP 1 346 717 wird Tetramethacryloxyethylpyrophosphat als Haftung vermittelnde Substanz beschrieben. Das Pyrophosphat soll unter den wässrigen Bedingungen aufbrechen und zu Phosphorsäureresten hydrolysieren, die anfänglich für einen sehr niedrigen pH-Wert sorgen sollen und helfen sollen, das Hydroxylapatit anzuätzen. Die Phosphorsäurereste sollen dann durch Calciumionen, die aus dem Dentin herauswandern sollen, neutralisiert werden und so einen zementartigen Haftverbund am Zahn bilden, während die Methacrylatgruppen durch Lichtpolymerisation mit dem Zahnfüllungsmaterial abreagieren können.

In der EP 1 721 949 A1 werden polymerisierbare Derivate der Ethylendiamintetraessigsäure als Haftvermittler in dentalen Adhäsivmaterialien vorgeschlagen. Der haftvermittelnde Effekt wurde dort am Beispiel des Di-Oxyethoxymethacrylsäure-ethylendiamintetraessigsäureesters gezeigt.

Härtbare Monomere umfassend ein polyalicyclisches Strukturelement sind grundsätzlich bekannt und werden in diversen Anwendungsgebieten, z.B. in der Dentaltechnik, eingesetzt.

EP 1 238 993 beschreibt ein Verfahren zur Herstellung von Acylharnstoff-Gruppen enthaltenden Polyisocyanaten sowie Gemische hiervon sowie deren Verwendung als Ausgangskomponente bei der Herstellung von Polyurethan-Kunststoffen.

EP 0 611 752 A1 offenbart ein Verfahren zur Herstellung von olefinisch ungesättigten, Urethangruppen aufweisenden Isocyanaten unter Einhaltung eines bestimmten NCO/OH-Äquivalentenverhältnisses. Die gemäß EP 0 611 752 A1 erhältlichen Isocyanate können als Bindemittel für bei Raumtemperatur einkomponentig zu verarbeitende Beschichtungsmaterialien eingesetzt werden.

EP 0 209 700 A2 und DE 35 22 005 beschreiben (Meth)acrylsäure-Derivate bestimmter Tricyclodecane mit zweibindigen Brückengliedern aus der Gruppe der Urethane bzw. Harnstoffe, welche im Dentalbereich eingesetzt werden können.

DE 10 2004 060 285 A1 betrifft strahlungshärtbare Zusammensetzungen auf Basis eines Dicidolgemisches (enthaltend zwei oder drei Isomere 3,8-, 4,8- und/oder 5,8-Bis(hydroxymethyl)tricyclo[5.2.1.0²,⁶]decane) mit mindestens einer Verbindung, welche mindestens eine ethylenisch ungesättigte Gruppierung mit gleichzeitig mindestens einer gegenüber Dicidol reaktiven Gruppierung aufweist, wobei diese Verbindung ein Umsetzungsprodukt aus Hydroxyalkyl(meth)acrylat und Diisocyanat sein kann. Die Zusammensetzungen gemäß DE 10 2004 060 285 A1 können verwendet werden als strahlungsinduziert härtende Beschichtungsstoffe, Klebstoffe, Laminierungen, Druckfarben und Tinten, Polituren, Lasuren, Pigmentpasten, Spachtelmassen, Kosmetikartikel, Verpackungsmaterialien und/oder Dicht- und/oder Dämmstoffe.

WO 2006/063891 A1 offenbart radikalisch polymerisierbare Verbindungen, im Wesentlichen enthaltend das Umsetzungsprodukt eines Dicidolgemisches und mindestens einer Verbindung, welche mindestens eine ethylenisch ungesättigte Gruppierung mit gleichzeitig mindestens einer gegenüber Dicidol reaktiven Gruppierung aufweist. Die Anwendungsgebiete entsprechen den in DE 10 2004 060 285 A1 genannten.

US 6,670,499 B1 beschreibt vom Adamantan abgeleitete Diurethane. Die in US 6,670,499 beschriebenen Verbindungen eignen sich als Intermediate für den Einsatz im dentalen Bereich oder zur Herstellung von optischen Materialien (wie beispielsweise Linsen).

US 6,794,528 B2 beschreibt Phosphorsäureester mit polialicyclischen Strukturelementen. Die in US 6,794,528 B2 offenbarten Verbindungen sind sehr hitzebeständig und zum Einsatz als Flammschutzmittel sowie als Weichmacher oder Stabilisatoren geeignet. Diese Verbindungen weisen insbesondere keine härtbaren Funktionalitäten auf.

JP 2007091642A offenbart Verbindungen der nachfolgenden Formeln

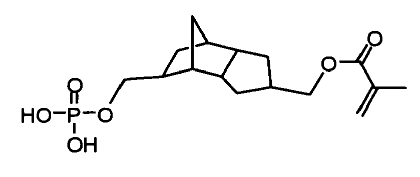

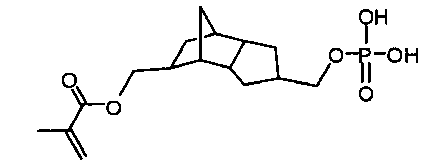

Die in JP 2007/091642A offenbarten Verbindungen, Erzeugnisse und Verfahren sind nicht Gegenstand der vorliegenden Erfindung.

DE 32 05 030 A1 offenbart Phosphatderivate und deren Verwendung zur Herstellung von Füllstoffen für beispielsweise Zähne. Manche der offenbarten Phosphatderivate umfassen eine Acryloyloxygruppe oder Methacryloyloxygruppe. Die in DE 32 05 030 A1 offenbarten Verbindungen, Erzeugnisse und Verfahren sind nicht Gegenstand der vorliegenden Erfindung.DE 603 12 714 T2 offenbart selbstätzende selbstgrundierende Einkomponenten-Dentalkleberzusammensetzungen umfassend ein polymerisierbares saures Phosphorsäureestermonomer einer Formel (A). Das Phosphorsäureestermonomer der Formel (A) kann in bestimmten Ausgestaltungen ausgewählte polyalicyclische Strukturelemente umfassen. US 2006/0246017 A1 offenbart in ähnlicher Weise ein polymerisierbares saures Phosphorsäureestermonomer einer Formel (A) zur Verwendung als selbstgrundierendes, selbstätzendes Adhäsiv. Die in DE 603 12 714 T2 und US 2006/0246017 A1 offenbarten Verbindungen, Erzeugnisse und Verfahren sind nicht Gegenstand der vorliegenden Erfindung.

Auf dem Gebiet der Dentaltechnik und für diverse andere Anwendungszwecke besteht ein ständiger Bedarf nach weiteren polymerisierbaren Monomeren. Insbesondere besteht ein Bedarf nach Monomeren, die die Herstellung von Erzeugnissen und Polymeren mit verbesserten Eigenschaften ermöglichen.

Primäre Aufgabe der vorliegenden Erfindung ist es, neue polymerisierbare Monomere anzugeben, die insbesondere für Anwendungen in der Dentaltechnik geeignet sind, ohne dabei auf dieses Einsatzgebiet beschränkt zu sein. Diese neuen polymerisierbaren Monomere sollen haftungsfördernde Monomere sein (auch als Haftvermittler bezeichnet), d.h. sie sollen ein gutes Haftvermögen auf Oberflächen aufweisen, vorzugsweise auf festen Oberflächen, insbesondere gegenüber Zahnhartsubstanz.

Dabei sollten diese Monomere in Erzeugnissen im ausgehärteten Zustand vorzugsweise ein besseres Haftvermögen auf einer Oberfläche, vorzugsweise gegenüber Zahnhartsubstanz, dabei insbesondere Dentin, aufweisen als Trimellitsäureanhydrid-4-methacryloyloxyethylester (4-META), weiter bevorzugt eine Haftkraft von zumindest 12,5 MPa.

Die adhäsive Verbindung von Kompositen mit Zahnhartmaterialien wird in der Regel durch die Verwendung des "Total-Etch" Verfahrens oder Primern erreicht, die die Zahnoberflächen für die Aufnahme von Adhäsivharzen vorbereiten. Die beim "Total-Etch" Verfahren eingesetzte, konzentrierte Phosphorsäure soll durch partielle Auflösung von Schmelz und Dentin und Öffnung der Dentinporen das Einfließen des Haftadhäsivs sicherstellen und durch das Schmelzätzmuster die retentive Verankerung erhöhen. Diese Ätzung mit Phosphorsäure ist ein zusätzlicher Arbeitsschritt bei der restaurativen Füllungstherapie, der sowohl aufwendig als auch relativ fehleranfällig ist. Zu langes wie auch zu kurzzeitiges Einwirkenlassen des Ätzmittels führt zu Problemen beim Haftverbund und unvollständige Entfernung bedingt häufig langandauernde pulpitische Beschwerden und Hypersensibilität. Andere Haftsysteme benötigen Primer, die die Kavitätenoberflächen modifizieren und vor der Anwendung des eigentlichen Adhäsivharzes zeitaufwendig verarbeitet werden müssen. Neuere Dentaladhäsive arbeiten nicht nach dem "Total-Etch" - Verfahren, sondern nach dem sogenannten "Non-Etch" - Verfahren und sind selbstkonditionierend. Solche Präparate sind wesentlich einfacher, anwendungssicherer und sparen bei der Behandlung Zeit.

Die neuen polymerisierbaren Monomere sollen vorzugsweise auch die Anwendung im Non-Etch Verfahren einsetzbar sein und die für das Bondingprozedere notwendige Dentinmodifizierung bewirken, d.h. ohne die Notwendigkeit eines separaten Ätzungsschrittes durch Säuren und ohne den Einsatz von Primern.

Die neuen polymerisierbaren Monomere sollen ferner eine haftfördernde Wirkung als Bestandteil von dentalen Füllungskompositen und Befestigungszementen, von Unterfüllungsmaterialien, von Flow-Materialien, von Fissurenversieglern, von Lacken, von Wurzelkanalmaterialien, von Stumpfaufbaumaterialien und von provisorischen Restaurationsmaterialien (Inlays, Onlays, Kronen, Brücken, Befestigungsmaterialien) entfalten.

Im Bereich der Dentaltechnik werden an dentale Polymere besondere Anforderungen gestellt, wie beispielsweise eine gute Bioverträglichkeit, eine geringe Toxizität des Monomeren (für den Fall, dass es nicht auspolymerisiert, sondern teilweise als Monomer in der Polymermatrix verbleibt), ein geringer Restmonomergehalt, etc..

Ferner sollten die Monomere vorzugsweise zudem eine hohe Biokompatibilität (d.h. Bioverträglichkeit) und hohe Beständigkeit aufweisen. Die gesuchten Monomere sollten ferner vorzugsweise einfach herzustellen und leicht zu verarbeiten sein.

Insbesondere aber sollen die unter Verwendung der erfindungsgemäßen Monomeren erhältlichen Polymere einen stabilen und hohen Haftverbund zwischen der Zahnhartsubstanz und dem dentalen Funktionswerkstoff herstellen und beispielsweise als Bonding im Falle eines Füllungskomposits einen randspaltfreien Verschluss der Kavität gewährleisten.

Diese Aufgaben werden gelöst durch eine Verbindung der Struktur

(HO)_{w}-P(=O)-[G-(L)ₓQ(YZ)_{b}]_{y},

wobei hier und nachfolgend gilt:
- jedes Q bedeutet unabhängig von etwaigen weiteren Gruppen Q ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei Q keinen weiteren Substituenten trägt oder einen oder mehrere Substituenten ausgewählt aus der Gruppe bestehend aus Alkylgruppen (dabei vorzugsweise Cl-C4-Alkyl), Alkoxygruppen (dabei vorzugsweise C1-C4-Alkoxy), Halogenatome (dabei vorzugsweise F) und Trifluormethylgruppen,
- der Index b ist eine natürliche Zahl ausgewählt aus der Gruppe der natürlichen Zahlen 1, 2 und 3,
- jedes Z bedeutet ein Strukturelement, das unabhängig von etwaigen weiteren Strukturelementen Z ausgewählt ist aus der Gruppe bestehend aus

   -(C=O)-CH=CH₂, -(C=O)-C(CH₃)=CH₂, und -CH=CH₂,
- jedes Y bedeutet ein Strukturelement, welches - unabhängig von etwaigen weiteren Strukturelementen Y - ein oder mehrere N-Atome enthält und das polyalicyclische Strukturelement Q mit Z so verbindet, dass in der Q mit Z verbindenden Kette von Atomen ein oder mehrere N-Atome enthalten sind,
- jedes Strukturelement G bedeutet - unabhängig von etwaigen weiteren Strukturelementen G - entweder O oder NH,
- jedes L bedeutet ein Strukturelement, welches - unabhängig von etwaigen weiteren Strukturelementen L - die Gruppe G mit dem polyalicyclischen Strukturelement Q verbindet,
- der Index w ist ausgewählt aus der Gruppe der natürlichen Zahlen 0, 1 und 2,
- jeder Index x bedeutet unabhängig von etwaigen weiteren Indizes x entweder 0 oder 1,
- der Index y ist ausgewählt aus der Gruppe der natürlichen Zahlen 1, 2 und 3,
wobei die Summe von w + y = 3 ist.

Die erfindungsgemäßen Monomere sind insbesondere für Anwendungen in der Zahnmedizin geeignet und weisen ein gutes Haftvermögen auf, insbesondere gegenüber Zahnhartsubstanz.

Die erfindungsgemäßen Monomere weisen im ausgehärteten Zustand in Erzeugnissen ein sehr gutes Haftvermögen auf festen Oberflächen auf, vorzugsweise gegenüber Dentin, dabei mit einer Haftkraft von zumindest 12,5 MPa.

Die erfindungsgemäßen Verbindungen sind als Additive für und als Bestandteil von haftverbessernde(n) Adhäsive(n) insbesondere für mineralische Untergründe und besonders Zahnhartmaterialien geeignet. Sie ermöglichen auch die Anwendung im Non-Etch Verfahren, da die erfindungsgemäßen Verbindungen, auch in Kombination mit einem oder mehreren weiteren Monomeren, offenbar die für das Bondingprozedere notwendige Dentinmodifizierung bewirken.

Weiterhin zeigen die erfindungsgemäßen Verbindungen eine haftverbessernde Wirkung als Bestandteil von dentalen Füllungskompositen und Befestigungszementen, von Unterfüllungsmaterialien, von Flow-Materialien, von Fissurenversieglern, von Lacken, von Wurzelkanalmaterialien, von Stumpfaufbaumaterialien und von provisorischen Restaurationsmaterialien (insbesondere Inlays, Onlays, Kronen, Brücken und/oder Befestigungsmaterialien).

In bevorzugten erfindungsgemäßen Verbindungen ist Y unabhängig von etwaigen weiteren Strukturelementen Y ausgewählt aus der Gruppe bestehend aus linearen, verzweigten oder Ringe umfassenden zweibindigen organischen Brückengliedern mit 1 bis 20 C-Atomen, vorzugsweise mit 1 bis 12 C-Atomen, bevorzugt mit 1 bis 8 C-Atomen, und gegebenenfalls 1 bis 6 Heteroatomen, vorzugsweise mit 1 bis 5 Heteroatomen, wobei die gegebenenfalls vorhandenen Heteroatome vorzugsweise gewählt sind aus der Gruppe bestehend aus N und O.

Bevorzugte erfindungsgemäße Verbindnungen weisen die folgende Struktur auf

(HO)_{w}-P(=O)-[G-(CH₂)ₖ-(A-C(=O))ᵣ(E)ⱼ-(CH₂)ₙ-Q(YZ)_{b}]_{y},

wobei gilt:
- jeder Index k ist eine natürliche Zahl, die unabhängig von etwaigen weiteren Indizes k ausgewählt ist aus der Gruppe der natürlichen Zahlen 0 bis 12, vorzugsweise aus der Gruppe der natürlichen Zahlen 0 bis 8,
- jedes Strukturelement A bedeutet - unabhängig von etwaigen weiteren Strukturelementen A - entweder O oder NH,
- jedes Strukturelement E bedeutet - unabhängig von etwaigen weiteren Strukturelementen E - entweder O oder NH,
- jeder Index r bedeutet unabhängig von etwaigen weiteren Indizes r entweder 0 oder 1,
- jeder Indexj bedeutet unabhängig von etwaigen weiteren Indizes j entweder 0 oder 1,
- jeder Index n bedeutet unabhängig von etwaigen weiteren Indizes n entweder 0 oder 1,
wobei wenn k = 0 ist, der Index j = 0 und der Index r = 0 bedeutet.
In einer bevorzugten Ausgestaltung bedeutet der Index k = 0 oder k = 2.
In einer bevorzugten Ausgestaltung bedeutet der Index n = 1.

In einer bevorzugten Ausgestaltung bedeutet das Strukturelement E Sauerstoff.

Für den Fall, dass y >1 ist, d.h. wenn y = 2 oder 3 ist, sind in einer bevorzugten Ausgestaltung die y Reste -[G-(CH₂)ₖ(A-C(=O)ME)ᵣ(CH₂)ₙQ(YZ)_{b}] einer erfindungsgemäßen Verbindung identisch.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", "tricyclisch", tetracyclisch", "pentacyclisch" und "hexacyclisch" entsprechen dabei der IUPAC-Nomenklatur.

Ein erfindungsgemäßes Monomer umfasst mindestens ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten YZ ersetzt sind (wie oben beschrieben), und optional ein, zwei oder mehr der nicht durch Substituenten YZ substituierten Wasserstoffatome durch Alkylgruppen, Alkoxygruppen, Halogenatome oder Trifluormethylgruppen substituiert sind.

Die Struktur unsubstituierter Bicyclen ist wie folgt: 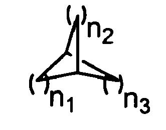 wobei n₁, n₂ und n₃ jeweils unabhängig voneinander eine natürliche Zahl von 1 bis 8 bedeuten können, vorzugsweise eine natürliche Zahl von 1 bis 4.

Exemplarisch genannt seien hier:

| | |
|---|---|
| Für n₁, = n₂ = 1; n₃ = 2 | Bicyclo[2.1.1]hexan |
| für n₁ =1; n₂ = n₃= 2 | Bicyclo[2.2.1 ]heptan |
| für n₁ = n₂ = 1; n₃ = 3 | Bicyclo[3.1.1]heptan |
| für n₁ = n₂ = n₃= 2 | Bicyclo[2.2.2]octan |
| für n₁ = n₂ = 1; n₃ = 4 | Bicyclo[4.1.1]octan |
| für n₁, =1; n₂ = 2; n₃= 3 | Bicyclo[3.2.1 ]octan |
| für n₁, =1; n₂ = 2; n₃= 4 | Bicyclo[4.2.1]nonan |
| für n₁ = n₂ = 2; n₃ = 4 | Bicyclo[4.2.2]decan |

Nachfolgend werden exemplarisch einige disubstituierte Bicyclen gezeigt: 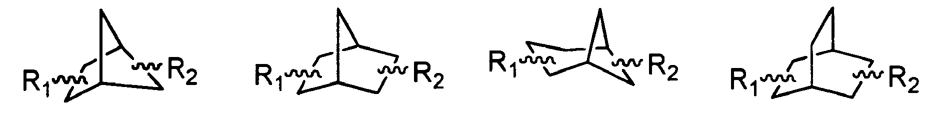 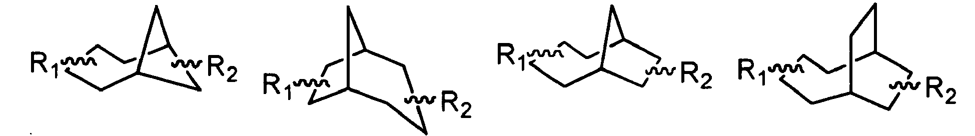 wobei R1 und R2 jeweils die sonstigen Reste der Verbindung bedeuten.

Beispiele für bicyclische Strukturelemente Q sind das Bicyclo[1.1.1]pentan-, das Bicyclo[2.1.1]hexan-, das Bicyclo[2.2.1]heptan-, das Bicyclo[3.1.1]heptan-, das Bicyclo[2.2.2]octan-, das Bicyclo[4.1.1]octan-, das Bicyclo[3.2.1]octan-, das Bicyclo[4.2.1]nonan-, das Bicyclo[3.3.1]nonan-, das Bicyclo[5.1.1]nonan-, das Bicyclo[3.2.2]nonan-, das Bicyclo[6.1.1]decan-, das Bicyclo[5.2.1]decan-, das Bicyclo[4.2.2]decan-, das Bicyclo[3.3.2]decan-, das Bicyclo[7.1.1]undecan-, das Bicyclo[6.2.1]undecan-, das Bicyclo[5.2.2]undecan-, das Bicyclo[4.3.2]undecan-, das Bicyclo[3.3.3]undecan-, das Bicyclo[8.1.1]dodecan-, das Bicyclo[7.2.1]dodecan-, das Bicyclo[6.2.2]dodecan-, das Bicyclo[5.3.2]dodecan-, das Bicyclo[4.3.3]dodecan-, das Bicyclo[4.4.2]dodecan-, das Bicyclo[5.4.1]dodecanstrukturelement sowie noch höhere Strukturelemente wie die entsprechenden Tridecane, Tetradecane, Pentadecane, etc.

Für unsubstituierte Tricyclen sind z.B. folgende Strukturen möglich: 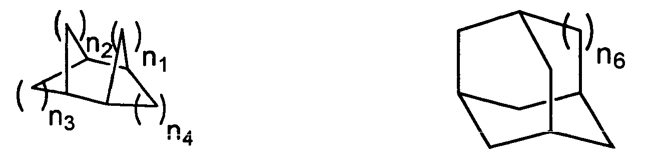 wobei n₁, n₂, n₃, n₄ bzw. n₆ jeweils unabhängig voneinander eine natürliche Zahl von 0 bis 5 bedeuten können.

Exemplarisch genannt seien:

| | |
|---|---|
| Für n₁ = 2; n₂ = 0; n₃= 2; n₄ = 3 | Tricyclo[4.3.2.0^{2,5}]undecan |
| für n₁ = 0; n₂ = 1; n₃= 2; n₄ = 3 | Tricyclo[5.2.1.0^{2,6}]decan |
| für n₁ = 0; n₂ = 2; n₃= 2; n₄ = 3 | Tricyclo[5.2.2.0^{2,6}]undecan |
| für n₁ = 2; n₂ = 0; n₃= 2; n₄ = 2 | Tricyclo[4.2.2.0^{2,5}]decan |
| für n₆ = 1 | Thcyclo[3.3.1.1^{3,7}]decan |

Nachfolgend werden exemplarisch einige di- oder trisubstituierte Tricyclen gezeigt: 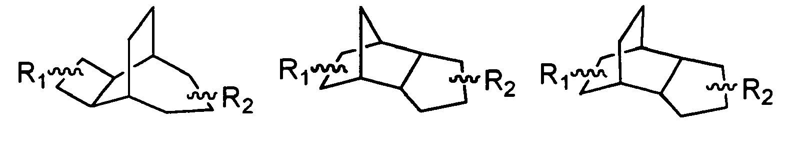 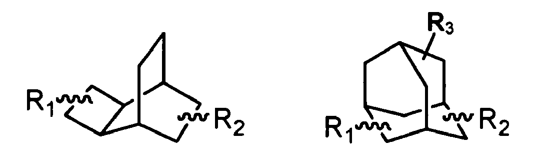 wobei R1, R2 und R3 jeweils die sonstigen Reste der Verbindung bedeuten.

Beispiele für tricyclische Strukturelemente Q sind das Thcyc!o[3.2.1.0^{2,6}]octan-, das Tricyclo[4.2.1.0^{2,6}]nonan-, das Tricyclo[5.2.1.0^{2,6}]decan-, das Tricyclo[6.2.1.0^{2,6}]undecan-, das Tricyclo[7.2.1.0^{2,6}]dodecan-, oder das Tricyclo[4.2.1.1^{2,5}]decan-, das Tricyclo[4.3.1.1 ^{2,5}]decan-, das Tricyclo[4.4.1.1 ^{2,5}]decan-, das Tricyclo[2.2.1.0^{2,6}]heptan-, das Tricyclo[2.2.2.0^{2,6}]octan-, das Tricyclo[3.2.2.0^{2,6}]nonan-, das Tricyclo[3.3.1.1^{3,7}]decan-, das Tricyclo[3.2.1.1^{3,7}]nonan-, das Tricyclo[4.2.2.2^{2,5}]dodecan-, das Tricyclo[4.3.2.2^{2,5}]tridecan-, das Tricyclo[4.4.2.2^{2,5}]tetradecan-, das Tricyclo[4.2.1.0 ^{3,7}]nonan-, das Tricyclo[4.4.1.1^{1,5}]dodecan-, das Tricyclo[6.2.1.0^{2,6}]undecan-, das Tricyclo[5.2.2.0^{2,6}]undecan, das Thcyc!o[6.2.2.0^{2,6}]dodecan-, Tricyclo[4.3.2.0^{2,6}]undecan-, dasTricyclo[4.2.2.0^{2,6}]decan- oder das Tricyclo[5.5.1.0 ^{3,11}]tridecanstrukturelement.

Für unsubstituierte Tetracyclen ist z.B. die folgende Struktur möglich: 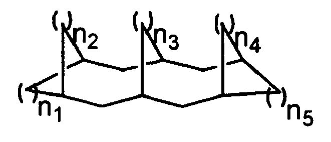 wobei n₁, n₂, n₃, n₄ und n₅ jeweils unabhängig voneinander eine natürliche Zahl von 1 bis 5 bedeuten können.

Exemplarisch genannt seien hier:

| | |
|---|---|
| Für n₁ = n₂ = n₃ = n₄ = 2; nₛ = 5 | Tetracyclo[9.6.2^{3,9}.2^{13,16}]tricosan |
| für n₁ = n₅ = 2; n₂ = n₃ = n₄ = 1 | Tetracyclo[6.6.1^{3,6}.1^{10,13}]heptadecan |
| für n₁ = n₂ = n₃ = n₄ = n₅ = 2 | Tetracyclo[6.6.2^{3,6}.2^{10,13}]eicosan |

Nachfolgend werden exemplarisch einige disubstituierte Tetracyclen gezeigt 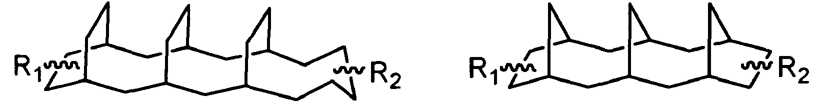 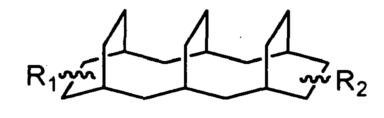 wobei R1 und R2 jeweils die sonstigen Reste der Verbindung bedeuten.

Beispiele für tetracyclische Strukturelemente Q sind das Tetracyclo[4.4.2.2 2,^{2,5}.1⁷,¹⁰]pentadecan-, das Tetracyclo[5.5.2.2^{2,6}.1^{8,12}]heptadecan-, das Tetracyclo[6.6.2.2^{2,7}.1^{9,14}]nonadecan-, das Tetracyclo[4.4.2.2^{2,5}.2^{7,10}]hexadecan-, das Tetracyclo[5.2.2^{2,6}.1^{8,11}] hexadecan-, das Tetracyclo[6.2.1.1^{3,6}.0^{2,7}]dodecan, das Tetracyclo[9.6.2^{3,9}.2^{13,16}]tricosan-, das Tetracyclo[9.6.2^{3,9}.2^{13,16}]tricosan-, das Tetracyclo[6.6.1^{3,6}.1^{10,13}]heptadecan-, das Tetracyclo[6.6.2^{3,6}.2^{10,13}]cosan-, oder das Tetracyclo[5.3.2.1^{2,4}.0^{3,6}]tridecanstrukturelement.

Beispiele für pentacyclische Strukturelemente Q sind das Pentacyclo[4.2.0.0^{2,5}.0^{3,8}.0^{4,7}]octan-, das Pentacyclo[13.7.4.3^{3,8}.0^{13,28}]thacontan-, das Pentacyclo[8.6.6.5^{2,9}.1^{23,26}]octacosan oder das Pentacyclo[3.3.0.0^{2,4}.0^{3,7}.0^{6,8}]octan-Strukturelement.

Ein Beispiel eines hexacyclischen Strukturelementes Q ist das Hexacyclo[15.3.2.2^{3,7}.1^{2,12}.0^{13,21}.0^{11,25}]pentacosan.

Bevorzugte Verbindungen, die zur Herstellung der erfindungsgemäßen Monomere geeignet sind, sind beispielsweise:
Alkoholsubstituierte polyalicyclische Kohlenwasserstoffe:
Bicyclo(2.2.1)heptan-2,7-diol,
[5-(Hydroxymethyl)-6-bicyclo[2.2.1]hept-2-enyl] -methanol,
Tricyclo[3.3.1.1^{3,7}]decan-1,3-diethanol,
Tetracyclo[6.3.0.0^{2,6}.0^{5,9}]undecan-3,11-diol,
[6-(Hydroxymethyl)-6-bicyclo[2.2.1]hept-2-enyl]methanol,
Tricyclo[3.3.^{13,7}]decan-1,3-diol,
Bis(hydroxymethyl)tricyclo[5.2.1.0^{2,6}]decan, etc..
Isocyanatsubstituierte polyalicyclische Kohlenwasserstoffe:
Bis(2-isocyanatoethyl)-5-norbornen-2,3-dicarboxylate,
2,5 (2,6)-Bis(isocyanatomethyl)bicyclo[2.2.1]heptan,
Bis(isocyanatomethyl)tricyclo[5.2.1.0^{2,6}]decan, etc..
Gemischt- oder Aminosubstituierte polyalicyclische Kohlenwasserstoffe:
Tricyclo[3.3.1.1^{3,7}]decan-1-ol-3-amino,
Pentacyclo[4.3.0.0^{2,5}.0^{3,8}.0^{4,7}]nonan-2,4-diamin,
Tricyclo[3.3.1.1^{3,7}]decan-1-ol-3-amino,
Bis(aminomethyl)tricyclo[5.2.1.0^{2,6}]decan, etc..

In einer bevorzugten Ausführungsform leitet sich die Struktur des polyalicyclischen Strukturelements Q von einem bicyclischen [a.c.d] Kohlenwasserstoff ab. Die Buchstaben a, c und d sind natürliche Zahlen und haben die Bedeutung der IUPAC-Nomenklatur. Die Summe von a, c und d liegt vorzugsweise im Bereich von 3 bis 13, bevorzugt im Bereich von 4 bis 7.

In einer weiteren bevorzugten Ausführungsform leitet sich die Struktur des polyalicyclischen Strukturelements von einem tricyclischen [a.c.d.f]-Kohlenwasserstoff ab. Die Summe von a, c, d und f liegt vorzugsweise im Bereich von 6 bis 12, bevorzugt im Bereich von 7 bis 9.

In einer bevorzugten Ausführungsform leitet sich die Struktur des polyalicyclischen Strukturelements von einem tricyclischen [a.2.1.0^{2,(a+1)}] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricayclischen[a.2.2.0^{2,(a+1)}] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricyclischen[a.3.1.1]Kohlenwasserstoff ab, wobei a jeweils die Zahl 3, 4, 5, 6 oder 7 bedeuten kann.

Bevorzugte erfindungsgemäße Verbindungen umfassen eine, zwei oder mehr funktionelle Gruppen ausgewählt aus der Gruppe bestehend aus
- Ester,
- Urethan,
- N-Acylurethan,
- Harnstoff,
- N-Acylharnstoff und
- Amid, wobei die Amidfunktion nicht unmittelbar mit einem N-Atom, einem O-Atom oder einer Carbonylgruppe verknüpft ist.

Hieraus ergibt sich, dass erfindungsgemäße Verbindungen, die eine spezielle Amidgruppe (wie definiert) enthalten, diese Amidgruppe beispielsweise nicht Bestandteil einer Urethan- oder Harnstoffgruppe ist.

Eine bevorzugte Amidgruppe ist (Meth)acrylamid.

Erfindungsgemäß bevorzugte Verbindungen sind dadurch gekennzeichnet, dass die Gruppierung YZ eine funktionelle Gruppe umfasst ausgewählt aus der Gruppe bestehend aus Ester, Urethan, N-Acylurethan, Harnstoff, N-Acylharnstoff und Amid, wobei die Amidfunktion nicht unmittelbar mit einem N-Atom, einem O-Atom oder einer Carbonylgruppe verknüpft ist.

Weiter erfindungsgemäß bevorzugte Verbindungen sind dadurch gekennzeichnet, dass die Gruppierung YZ eine funktionelle Gruppe umfasst ausgewählt aus der Gruppe bestehend aus Ester, Urethan, N-Acylurethan, Harnstoff, N-Acylharnstoft und Methacrylamid.Vorzugsweise umfasst hierbei die Gruppierung YZ eine funktionelle Gruppe ausgewählt aus der Gruppe bestehend aus Urethan, N-Acylurethan, Harnstoff, N-Acylharnstoff und Methacrylamid, wobei das bzw. zumindest eines der N-Atome dieser funktionellen Gruppe sich in der Q mit Z verbindenden Kette von Atomen befindet.

Bevorzugte erfindungsgemäße Verbindungen sind solche, die keine Carbonat-Gruppe enthalten, insbesondere solche, in denen das Strukturelement E und das Strukturelement A nicht beide gleichzeitig Sauerstoff bedeuten.

Bevorzugt sind erfindungsgemäße Verbindungen, in denen
(i) das Strukturelement Z = -(C=O)-C(CH₃)=CH₂ bedeutet,
   und/oder
(ii) die funktionellen Gruppen Ester, Urethan-, Harnstoff- oder Methacrylamid-Gruppen
   sind, da mit diesen Verbindungen besonders gute Ergebnisse erzielt wurden, und/oder
(iii) das Strukturelement Q einen Tricyclo[5.2.1.0^{2,6}]decan-Rest bedeutet.

Weiter bevorzugt sind erfindungsgemäße Verbindungen, in denen das Strukturelement Z = -(C=O)-C(CH₃)=CH₂ bedeutet, wobei die funktionellen Gruppen Ester, Urethan-, Harnstoff- oder Methacrylamid-Gruppen sind und das Strukturelement Q einen Tricyclo[5.2.1.0^{2,6}]decan-Rest bedeutet.

Bevorzugt sind erfindungsgemäße Verbindungen, in denen sämtliche vorhandenen lichthärtbaren Gruppen dem Strukturelement Z entsprechen.

Eine erfindungsgemäße Verbindung kann neben lichthärtbaren Gruppen des Strukturelements Z auch weitere polymerisierbare, vorzugsweise endständige polymerisierbare, Gruppen umfassen, die nicht lichthärtbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.Weiter bevorzugte Verbindungen sind dadurch gekennzeichnet, dass die Gruppierung YZ eine Struktur aufweist ausgewählt aus der Gruppe bestehend aus
(-CH₂)ₙ-O-C(=O)-NH-Z,
(-CH₂)ₙ-O-C(=O)-NH-(CH₂)ₘ₋O-Z,
(-CH₂)ₙ-NH-C(=O)-NH-Z,
(-CH₂)ₙ-NH-C(=O)-NH-(CH₂)ₘ-O-Z,
(-CH₂)ₙ-NH-Z,
und
(-CH₂)ₙ-NH-C(=O)-O-(CH₂)ₘ-O-Z,
wobei Z und n die oben angegebene Bedeutung haben und wobei gilt:
- jeder Index m ist eine natürliche Zahl, die unabhängig von etwaigen weiteren Indizes m ausgewählt ist aus der Gruppe der natürlichen Zahlen 1 bis 12, vorzugsweise aus der Gruppe der natürlichen Zahlen 2 bis 8.

In einer bevorzugten Ausgestaltung bedeutet der Index n = 1.

In einer bevorzugten Ausgestaltung bedeutet der Index m = 2.

Bevorzugte erfindungsgemäße Verbindungen sind solche, wobei Q ein polyalicyclisches Strukturelement bedeutet, vorzugsweise ein gesättigtes polyalicyclisches Strukturelement, das ausgewählt ist aus der Gruppe bestehend aus bicyclischen oder tricyclischen Kohlenwasserstoffresten, wobei vorzugsweise keines der nicht bereits durch Substituenten YZ oder durch die jeweilige das Strukturelement G-(L)ₓ umfassende Gruppe substituierten Wasserstoffatome dieses polyalicyclischen Strukturelements Q substituiert ist.

Ein bevorzugter bicyclischer Kohlenwasserstoffrest ist ein Bicyclo[2.2.1]heptan-Rest, d.h. bevorzugt sind erfindungsgemäße Verbindungen, die ein Bicyclo[2.2.1]heptan (Norbornan) - Gerüst aufweisen.

In einer weiteren bevorzugten Ausführungsform leitet sich die Struktur des polyalicyclischen Strukturelements Q von einem Tricyclodecan- bzw. Tricyclodecen-Kohlenwasserstoffrest ab.

Besonders bevorzugt sind erfindungsgemäße Monomere, deren polyalicyclisches Strukturelement Q sich von einem der folgenden tricyclischen Kohlenwasserstoffe ableitet: Tricyclo[5.2.1.0^{2,6}]decan (TCD), Tricyclo[5.2.1.0^{2,6}]dec-3-en oder Tricyclo[3.3.1.1^{3,7}]decan (Adamantan), d.h. bevorzugt sind erfindungsgemäße Verbindungen, die ein TCD - Gerüst, ein Tricyclo[5.2.1.0^{2,6}]dec-3-en - Gerüst oder ein Adamantan-Gerüst aufweisen.

Besonders bevorzugte erfindungsgemäße Verbindungen sind solche, wobei das Strukturelement Q einen Tricyclo[5.2.1.0^{2,6}]decan-Rest, einen Tricyclo[5.2.1.0^{2,6}]dec-3-en-Rest, einen Tricyclo[3.3.1.1³,⁷]decan-Rest oder einen Bicyclo[2.2.1]heptan-Rest bedeutet.

Bei den genannten besonders bevorzugten erfindungsgemäßen Verbindungen, in denen das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet, handelt es sich vorzugsweise um solche mit einem Tricyclo[5.2.1.0^{2,6}]decan-Gerüst, einem Tricyclo[5.2.1.0^{2,6}]dec-3-en-Gerüst, einem Tricyclo[3.3.1.1^{3,7}]decan-Gerüst bzw. einem Bicyclo[2.2.1]heptan-Gerüst, bei dem jeweils keines der nicht durch Substituenten YZ substituierten Wasserstoffatome dieses polyalicyclischen Strukturelements Q substituiert ist.

Die vorliegende Erfindung betrifft ferner ein Verfahren zur Herstellung einer Verbindung der Struktur (HO)_{w}P(=O)-[G-(L)ₓQ(YZ)_{b}]_{y} oder einer Mischung umfassend zumindest eine Verbindung der Struktur (HO)_{w}-P(=O)-[G-(L)ₓ-Q(YZ)_{b}]_{y}, jeweils vorzugsweise in einer der oben genannten bevorzugten bzw. besonders bevorzugten Ausgestaltungen, mit folgenden Schritten:
(i) Bereitstellen einer Verbindung HG-(L),-Q(YZ)_{b},
(ii) Umsetzung der Verbindung aus Schritt (i) mit POCl₃ oder Phosphor(V)-oxid, vorzugsweise in Gegenwart eines Amins, bevorzugt eines tertiären Amins,
(iii) Hydrolyse des in Schritt (ii) gebildeten Umsetzungsproduktes,
wobei G, L, Q, Y, Z, w, x, b und y jeweils die oben genannte Bedeutung haben, und wobei die molare Menge der Verbindung aus Schritt (i) pro Atomäquivalent Phosphor im Bereich von 0,5 bis 6 liegt, vorzugsweise im Bereich von 0,6 bis 5, bevorzugt im Bereich von 0,7 bis 4, weiter bevorzugt im Bereich von 0,8 bis 3,5.

Phosphor(V)-oxid ist auch unter dem Namen Phosphorpentoxid bekannt und wird häufig mit der Formel P₂O₅ angegeben, wobei Phosphor(V)-oxid exakter durch die Formel P₄O₁₀ (Diphosphorpentoxid) beschrieben wird.

Die Herstellung der erfindungsgemäßen Verbindungen wird beispielhaft anhand der folgenden Reaktionsschemata erläutert.

Die darin angegebenen Verfahrensschritte 1.) ii) bzw. 2.) iii) beziehen sich dabei auf die Schritte ii) (Umsetzung der Verbindung aus Schritt (i) mit POC1₃ oder Phosphor(V)-oxid) bzw. iii) (Hydrolyse des in Schritt (ii) gebildeten Umsetzungsproduktes) eines erfindungsgemäßen Herstellverfahrens.

Dabei gilt in Schema 1 und Schema 2 jeweils:
Q, n, Z, G, k, w und y haben die oben angegebene Bedeutung,
R ist ein Strukturelement, das die OCN-Gruppe mit dem Strukturelement Z verbindet, vorzugsweise ist R eine Gruppe -(CH₂)ₘ-O, wobei m die oben genannte Bedeutung hat,
der Index q bedeutet unabhängig von etwaigen weiteren Inidizes q enweder 0 oder 1. 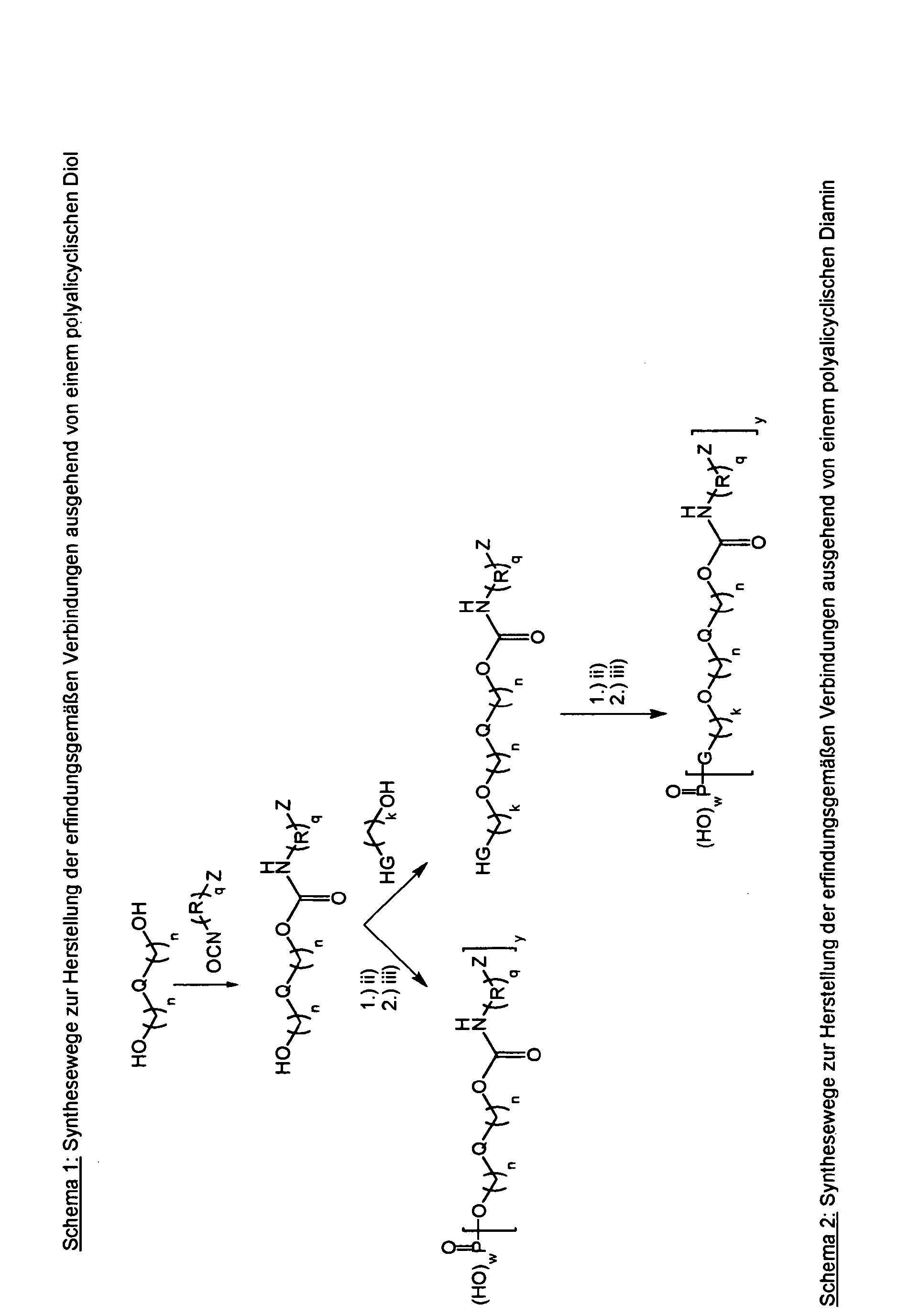 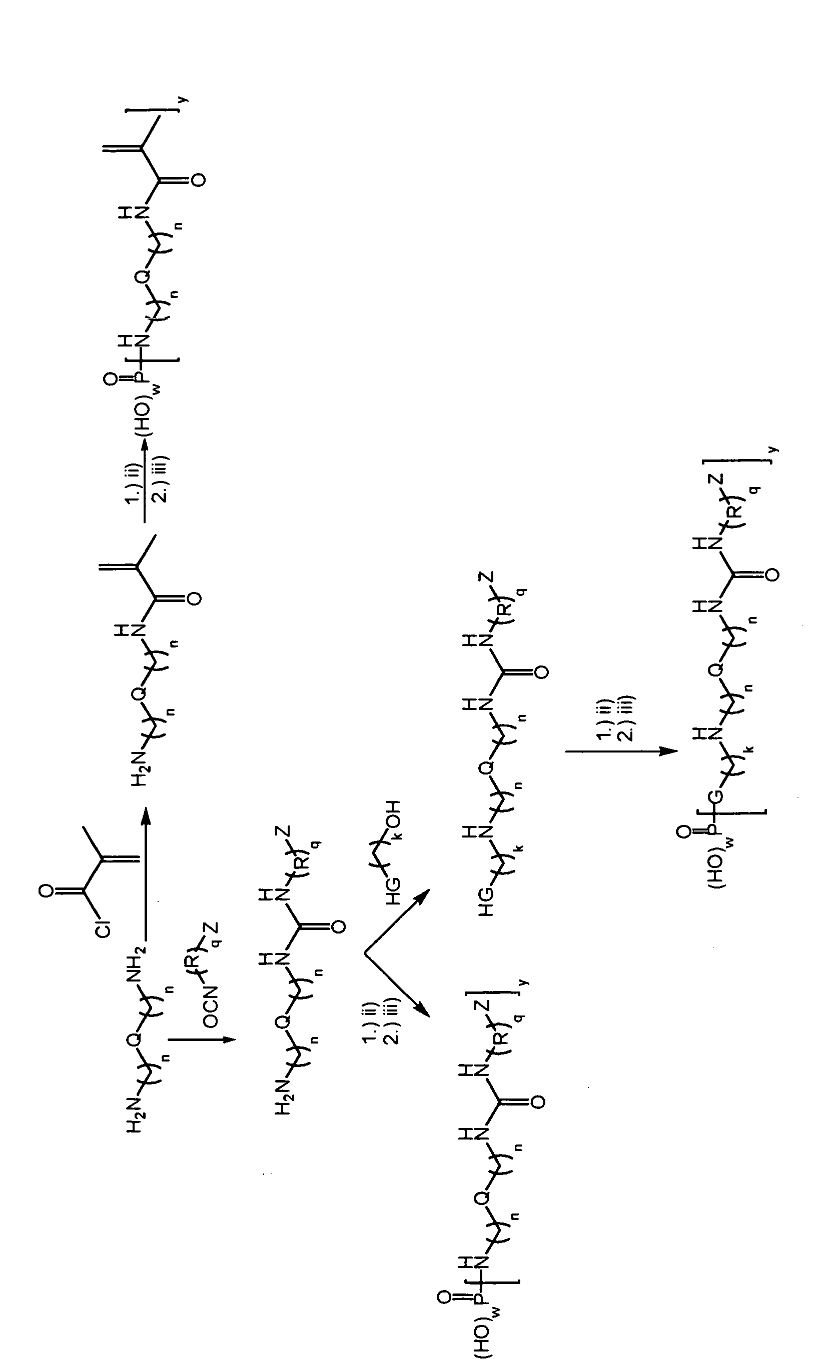 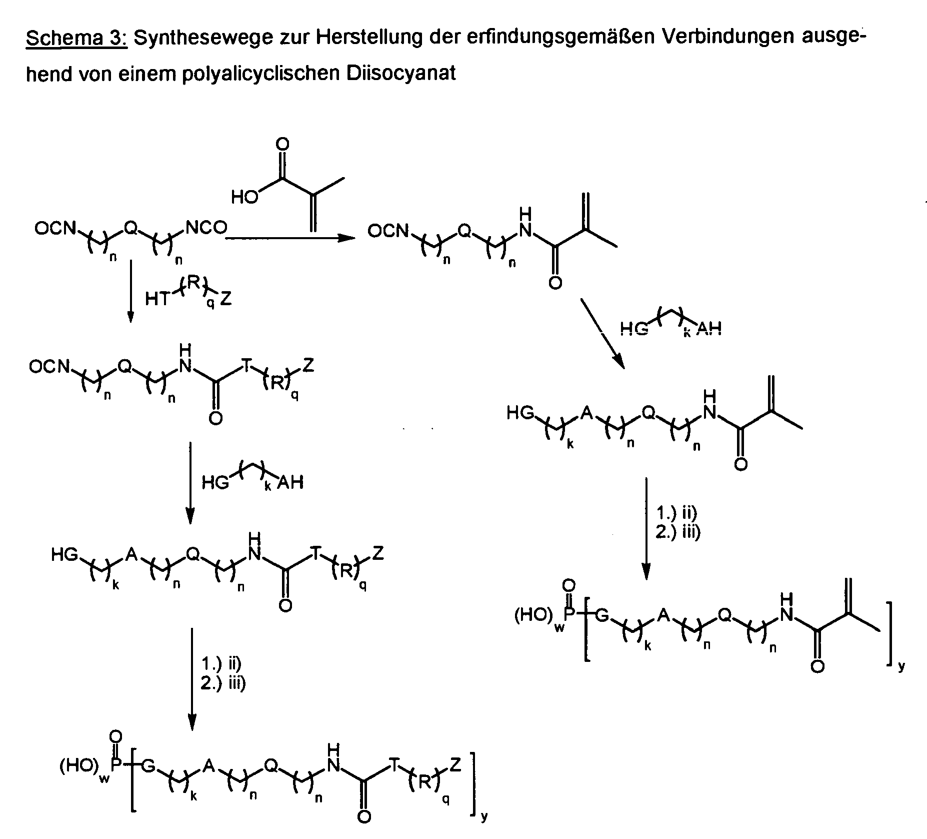
wobei Q, n, Z, G, A, k, w, y, R und q die oben angegebene Bedeutung haben und
jedes Strukturelement T - unabhängig von etwaigen weiteren Strukturelementen T - entweder O oder NH bedeutet.

Die vorstehenden bzw. nachfolgenden Ausführungen zu den als bevorzugt und besonders bevorzugt gekennzeichneten erfindungsgemäßen Verbindungen gelten für die bevorzugten und besonders bevorzugten Ausgestaltungen der erfindungsgemäßen Verfahren, Mischungen, Gemische, Erzeugnisse und Verwendungen jeweils entsprechend.

In einem weiteren Aspekt betrifft die vorliegende Erfindung eine Mischung umfassend eine, zwei oder mehr unterschiedliche erfindungsgemäße Verbindungen, vorzugsweise herstellbar nach einem erfindungsgemäßen Verfahren.

In einem weiteren Aspekt betrifft die vorliegende Erfindung ein härtbares Gemisch, umfassend
(a) eine oder mehrere erfindungsgemäße Verbindungen oder eine erfindungsgemäße Mischung
   und
(b) einen oder mehrere weitere Bestandteile gewählt aus der Gruppe bestehend aus
   (b-1) von Bestandteil (a) verschiedene Monomere, welche mit Bestandteil (a) co-polymerisierbar sind, vorzugsweise lichtpolymerisierbare Monomere,
   (b-2) einen oder mehrere Füllstoffe, vorzugsweise einen oder mehrere nanoskalige Füllstoffe,
   (b-3) Photoinitiatoren und Initiatoren für die chemische Aushärtung,
   (b-4) Polymerisationsinhibitoren,
   (b-5) Lösungsmittel,
   und
   (b-6) von Bestandteil (a) verschiedene haftfördernde Additive.

Bei einem bevorzugten erfindungsgemäßen Gemisch handelt es sich um eine chemisch und/oder durch Licht induziert und/oder thermisch induziert härtbare dentale Zusammensetzung.

Bevorzugt liegt die Gesamtmasse der erfindungsgemäßen Verbindungen der Komponente (a) im Bereich von 0,1 bis 50 Gew.-%, bevorzugt im Bereich von 5 bis 40 Gew.-%, weiter bevorzugt im Bereich von 10 bis 35 Gew.-%, jeweils bezogen auf die Gesamtmasse des Gemisches.

### Bestandteil (b-1) - polymerisierbare Monomere

Die polymerisierbare Monomere sind vorzugsweise radikalisch lichtpolymerisierbare Monomere, die bevorzugt eine, zwei oder mehr ethylenische Gruppen aufweisende Substanzen sind wie beispielsweise, ohne darauf beschränkt zu sein, die in der Dentalchemie üblicherweise eingesetzten (Meth)acrylatmonomere.

In der Patentliteratur ist eine Vielzahl weiterer Verbindungen genannt (beispielsweise auch in der DE 39 41 629 A1, welche im Wege der Verweisung Bestandteil der vorliegenden Anmeldung ist), die allesamt Diester der Acryl- oder Methacrylsäure sind und sich zum Einsatz in einem erfindungsgemäßen härtbaren Gemisch eignen.

In einem bevorzugten erfindungsgemäßen härtbaren Gemisch enthält Bestandteil (b-1) ein oder mehrere Dimethacrylat-Monomere gewählt aus der Gruppe bestehend aus Ethylenglykoldimethacrylat, 1,6-Hexandioldimethacrylat (HEDMA), Triethylenglykoldimethacrylat (TEDMA), 1,12-Dodecandioldimethacrylat, ethoxyliertes Bisphenol-A-dimethacrylat, Polyethylenglykoldimethacrylat, 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA) und Dimethacrylaten des Dihydroxymethyltricyclo[5.2.1.0^{2,6}]decans.

Die radikalisch lichtpolymerisierbaren Monomere können auch mindestens eine ethylenische Doppelbindung aufweisende Hydroxylverbindungen sein. Dabei können vorzugsweise die in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Weiterhin können auch lichthärtbare Monomere mit ethylenischen Doppelbindungen auf Basis von Polysiloxanen, wie sie beispielsweise in der DE 199 03 177 oder in der DE 44 16 857 beschrieben sind, verwendet werden, welche im Wege der Verweisung Bestandteil der vorliegenden Anmeldung sind.

Ein erfindungsgemäß bevorzugtes Gemisch ist dadurch gekennzeichnet, dass Bestandteil (b-1) besteht aus oder umfasst
(b-1a) ein oder mehrere (Meth)acrylat-Monomere, vorzugsweise ausgewählt aus der Gruppe bestehend aus 2-Hydroxyethylmethacrylat (HEMA), Bisphenol-A-glycidyl-methacrylat (Bis-GMA), 7,7,9-Trimethyl-4,13-dioxo-5,12-diazahexadecan-1,16-dioxydimethacrylat (UDMA), Triethylenglycoldimethacrylat (TEDMA), Tetraethylenglycoldimethacrylat, Polyethylenglykoldimethacrylat, Trimethylolpropantrimethacrylat (TMPTMA), Dodecandioldimethacrylat (DODMA), Glycerindi(meth)acrylat, 1,6-Hexandioldimethacrylat (HEDMA), ethoxyliertes Bisphenol-A-dimethacrylat, Pentaerythritoldimethacrylat, Pentaerythritol-tri(meth)acrylat und Dipentaerythritolpenta(meth)acrylat,
   und/oder
(b-1 b) ein oder mehrere Monomere ausgewählt aus der Gruppe bestehend aus Verbindungen der Struktur Q^{a}(MXₑ)ₕ, wobei gilt:
   - Q^{a} ist ein polyalicyclisches Strukturelement und hat - unabhängig von der Bedeutung des Strukturelements Q in Verbindungen des Bestandteils (a) - die oben für Q angegebenen Bedeutung,
   - h ist eine natürliche Zahl ausgewählt aus der Gruppe der natürlichen Zahlen 1, 2, 3 und 4,
   - jedes X bedeutet ein Strukturelement, das - unabhängig von etwaigen weiteren Strukturelementen X - ausgewählt ist aus der Gruppe bestehend aus
      -O-(C=O)-CH=CH₂, -O-(C=O)-C(CH₃)=CH₂,
      -(C=O)-CH=CH₂, -(C=O)-C(CH₃)=CH₂ und -O-CH=CH₂,
- jeder Index e ist eine natürliche Zahl, die unabhängig von etwaigen weiteren Indizes e, ausgewählt ist aus der Gruppe der natürlichen Zahlen 1, 2, 3 und 4,
- jedes M bedeutet ein Strukturelement, welches - unabhängig von etwaigen weiteren Strukturelementen M - in der Struktur Q^{a}(MXₑ)ₕ das polyalicyclische Strukturelement Q^{a} mit e Strukturelementen X verbindet.

Bevorzugt liegt die Gesamtmasse der Monomere der Komponente (b-1) im Bereich von 0,1 bis 60 Gew.-%, bevorzugt im Bereich von 10 bis 50 Gew.-%, weiter bevorzugt im Bereich von 15 bis 40 Gew.-%, jeweils bezogen auf die Gesamtmasse des Gemisches. Vorzugsweise enthält ein erfindungsgemäßes Gemisch zwei oder mehrere Monomere der Komponente (b-1), wobei vorzugsweise mindestens ein Monomer der Komponente (b-1 b) enthalten ist.

Bevorzugte erfindungegemäße Gemische sind dadurch gekennzeichnet, dass Komponente (b-1a) ein oder mehrere (Meth)acrylat-Monomere enthalten ausgewählt aus der Gruppe bestehend aus 2-Hydroxyethylmethacrylat (HEMA), Bisphenol-A-glycidyl-methacrylat (Bis-GMA), 7,7,9-Trimethyl-4,13-dioxo-5,12-diazahexadecan-1,16-dioxydimethacrylat (UDMA), Triethylenglycoldimethacrylat (TEDMA), Tetraethylenglycoldimethacrylat, Trimethylolpropantrimethacrylat (TMPTMA), Glycerindi(meth)acrylat, 1,6-Hexandioldimethacrylat (HEDMA), ethoxyliertes Bisphenol-A-dimethacrylat und Dipentaerythritolpenta(meth)acrylat und deren Mischungen.

Bevorzugte erfindungegemäße Gemische sind dadurch gekennzeichnet, dass Komponente (b-1 b) ein oder mehrere (Meth)acrylat-Monomere der Struktur Q^{a}(MXₑ)ₕ enthalten, da die Monomere der Komponente (b-1 b) im Kombination mit den erfindungsgemäßen Monomeren der Komponente (a) eine weitere Verbesserung der Eigenschaften bewirken, unter anderem eine weiter verbesserte Haftwirkung.

Es wurde zudem gefunden, dass Monomere der obigen Struktur Q^{a}(MXₑ)ₕ, vorzugsweise (Meth)acrylat-Monomere der Struktur Q^{a}(MXₑ)ₕ die Haftfestigkeit von Haftmonomeren, insbesondere der im Rahmen des vorliegenden Textes als bevorzugt gekennzeichneten Haftmonomere, erhöhen.

Daher betrifft die Erfindung in einem weiteren Aspekt die Verwendung von (Meth)acrylatMonomeren der obigen Struktur Q^{a}(MXₑ)ₕ zur Erhöhung der Haftungseigenschaften, d.h. zur Steigerung der Haftkraft, von Haftmonomeren.

Dies gilt insbesondere auch für Bis(methacryloyloxymethyl)tricyclo[5.2.1.0^{2,6}]decan und/oder Bis(acryloyloxymethyl)tricyclo[5.2.1.0^{2,6}]decan.

Ein erfindungsgemäß bevorzugtes Gemisch ist daher dadurch gekennzeichnet, dass Komponente (b-1 b) umfasst oder besteht aus
Bis(methacryloyloxymethyl)tricyclo[5.2.1.0^{2,6}]decan und/oder
Bis(acryloyloxymethyl)tricyclo[5.2.1.0^{2,6}]decan.

Ebenfalls bevorzugte Monomere der Struktur Q^{a}(MXₑ)ₕ der Komponente (b-1 b) sind in DE 10 2010 041 792.0 beschrieben.

Die bevorzugten Monomere der Struktur Q^{a}(MXₑ)ₕ sind solche, wobei das Strukturelement Q^{a} einen Tricyclo[5.2.1.0^{2,6}]decan-Rest, einen Tricycio[3.3.1.1^{3,7}]decan-Rest oder einen Bicyclo[2.2.1]heptan-Rest bedeutet.

Bevorzugte Monomere der Struktur Q^{a}(MXₑ)ₕ sind solche mit zwei, drei, vier oder mehr funktionellen Gruppen ausgewählt aus der Gruppe bestehend aus N-Acylharnstoff, Allophanat, Biuret und (Meth)acrylamid.

Ferner bevorzugt sind Monomere der Struktur Q^{a}(MXₑ)ₕ, in denen h 2 oder 3 bedeutet.

Bevorzugt sind Monomere der Struktur Q^{a}(MXₑ)ₕ der Komponente (b-1 b), in denen
(i) das Strukturelement X = -O-(C=O)-C(CH₃)=CH₂ bedeutet, wobei die funktionellen Gruppen Allophanat-, Biuret- oder Acylharnstoff-Gruppen sind, da mit diesen Verbindungen besonders gute Ergebnisse erzielt wurden,
   und/oder
(ii) das Strukturelement Q^{a} einen Tricyclo[5.2.1.0^{2,6}]decan-Rest bedeutet.

Weiter Bevorzugt sind erfindungsgemäße Verbindungen, in denen das Strukturelement X = -O-(C=O)-C(CH₃)=CH₂ bedeutet, wobei die funktionellen Gruppen Allophanat-, BiuretoderAcylharnstoff-Gruppen sind und das Strukturelement Q^{a} einen Tricyclo[5.2.1.0^{2,6}]decan-Rest bedeutet.

### Bestandteil (b-2) - Füllstoffe

Als Bestandteil (b-2) können organische und/oder anorganische Füllstoffe eingesetzt werden.

Sofern ein erfindungsgemäßes Gemisch einen oder mehrere Füllstoffe der Komponente (b-2) enthält, liegt die Gesamtmasse der Füllstoffe vorzugsweise im Bereich von 0,5 bis 75 Gew.-%, bevorzugt im Bereich von 10 bis 70 Gew.-%, weiter bevorzugt im Bereich von 30 bis 65 Gew.-%, jeweils bezogen auf die Gesamtmasse des Gemisches.

Anorganische Füllstoffe können allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen. Die mittlere Partikelgröße d₅₀ der erfindungsgemäß einzusetzenden Füllstoffpartikel der Füllstoffkomponente (b-2) eines erfindungsgemäßen Gemisches wird mittels Lichtstreuung (Laserbeugung) bestimmt, vorzugsweise mit einem Partikelgrößenmessgerät Beckman Coulter LS 13320.

Die Füllstoffe der Komponente (b-2) werden in Abhängigkeit vom Verwendungszweck des jeweils erfindungsgemäßen Dentalwerkstoffs, der die erfindungsgemäßen Verbindungen enthält, ausgwählt.

So werden die Füllstoffe in erfindungsgemäßen Gemischen, beispielsweise für dentale Zemente wie Befestigungszemente, vorzugsweise in Form von Mikropartikeln mit einer mittleren Partikelgröße im Bereich von 0,4 µm bis 10 µm eingesetzt.

Unter Mikropartikeln werden in Zusammenhang mit der vorliegenden Erfindung Partikel mit einer mittleren Partikelgröße von 400 nm bis 10 µm verstanden. Vorzugsweise ist die mittlere Partikelgröße kleiner als 5 µm.

Die Mikropartikel der Komponente (b-2) können dabei eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden.

Bevorzugt wird somit in einem erfindungsgemäßen Gemisch, beispielsweise in einem dentalen Zement, eine Komponente (b-2) eingesetzt, welche zwei oder mehr Fraktionen von Mikropartikeln enthält, wobei sich die mittleren Partikelgrößen der Fraktionen unterscheiden.

Vorzugsweise enthält Komponente (b-2) zumindest zwei Mikropartikel-Fraktionen, wobei deren mittlere Partikelgrößen um mindestens 0,5 µm, bevorzugt um mindestens 0,7 µm voneinander abweichen.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Bevorzugt umfasst ein erfindungsgemäßes Gemisch, beispielsweise ein dentaler Zement, eine Komponente (b-2), welche eine oder mehrere erste Mikropartikelfraktionen, die jeweils eine mittlere Partikelgröße im Bereich von 1 µm bis 10 µm, vorzugsweise 1 µm bis 5 µm, besitzen, und eine oder mehrere zweite Mikropartikelfraktionen, die jeweils eine mittlere Partikelgröße im Bereich von > 0,4 µm bis < 1 µm (d.h. größer als 0,4 µm, aber kleiner als 1 µm), vorzugsweise im Bereich von 0,5 µm bis 0,8 µm, besitzen.

Vorzugsweise liegt das Verhältnis der Gesamtmasse der ersten Mikropartikelfraktionen zur Gesamtmasse der zweiten Mikropartikelfraktionen im Bereich von 5 : 1 bis 1 : 2, bevorzugt im Bereich von 4 : 1 bis 2 : 3, weiter bevorzugt im Bereich von 3 : 1 bis 1 : 1.

Bevorzugt liegt das Verhältnis der mittleren Korngröße der oder einer ersten Mikropartikelfraktion zur mittleren Korngröße der oder einer zweiten Mikropartikelfraktion der Komponente (b-2) im Bereich von 1,5 : 1 bis 12 : 1, vorzugsweise im Bereich von 2 : 1 bis7:1.

In einem besonders bevorzugten erfindungsgemäßen Gemisch, beispielsweise in einem dentalen Zement, umfasst die Komponente (b-2) eine oder mehrere erste Mikropartikelfraktionen, die jeweils eine mittlere Partikelgröße im Bereich von 1 µm bis 10 µm, vorzugsweise 1 µm bis 5 µm, besitzen, und eine oder mehrere zweite Mikropartikelfraktionen, die jeweils eine mittlere Partikelgröße im Bereich von > 0,4 µm bis < 1 µm, vorzugsweise im Bereich von 0,5 µm bis 0,8 µm, besitzen; wobei das Verhältnis der Gesamtmasse der ersten Mikropartikelfraktionen zur Gesamtmasse der zweiten Mikropartikelfraktionen im Bereich von 5 : 1 bis 1 : 2, vorzugsweise im Bereich von 4 : 1 bis 2 : 3 und/oder das Verhältnis der mittleren Korngröße der oder einer ersten Mikropartikelfraktion zur mittleren Korngröße der oder einer zweiten Mikropartikelfraktion der Komponente (b-2) im Bereich von 1,5 : 1 bis 12 : 1, vorzugsweise 2 : 1 bis 7 : 1 liegt.

Zur besseren Einbindung in die Polymermatrix einer erfindungsgemäßen Zusammensetzung können die Mikropartikel organisch oberflächenmodifiziert sein. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders Methacryloxypropyltrimethoxysilan.

Ein erfindungsgemäßes Mikropartikel enthaltendes Gemisch, beispielsweise ein dentaler Zement, kann zusätzlich auch nanoskalige Füllstoffe enthalten.

Als anorganische Füllstoffe kommen beispielsweise kompakte Gläser und unterschiedliche Kieselsäuren in verschiedenen Größen und Zuständen (monodispers, polydispers) zum Einsatz.

Geeignete anorganische Bestandteile sind beispielsweise amorphe Materialien auf der Basis von Mischoxiden aus SiO₂, ZrO₂ und/oder TiO₂, mikrofeine Füllstoffe wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/AI-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Bevorzugte röntgenopake Füllstoffe sind ausgewählt aus der Gruppe bestehend aus Zink, Ytterbium, Yttrium, Zirkonium, Strontium, Calcium, Titan, Wolfram, Tantal, Niob, Barium, Wismut, Molybdän in Form von Legierungen, Oxiden, Fluoriden, Oxohalogeniden, Sulfaten, Phosphaten, Silikaten, Carbonaten, Wolframaten oder Gläsern und deren Mischungen.

Vorteilhafte röntgenopake Füllstoffe sind dabei CaWO₄, ZrO₂, Ytterbiumfluorid, Bariumsulfat und/oder röntgenopake Gläser.

Zur Einstellung der Rheologie können erfindungsgemäße härtbare Gemische und Erzeugnisse unterschiedliche Kieselsäuren, bevorzugt pyrogene Kieselsäuren, enthalten.

Daneben können verstärkend wirkende Materialien wie Glasfasern, Polyamid- oder Kohlenstofffasern eingesetzt werden. Die erfindungsgemäßen härtbaren Gemische und Erzeugnisse können zusätzlich feinteilige Splitter oder Perlpolymerisate enthalten, wobei die Perlpolymerisate Homo- oder Copolymere organischer härtbarer Monomerer sein können. Die organischen Füllstoffe sind grundsätzlich in unterschiedlicher Körnung anwendbar, wie zum Beispiel aufgemahlene Polymere und Präpolymere.

Ebenfalls bevorzugt enthalten die erfindungsgemäßen härtbaren Gemische und Erzeugnisse, insbesondere zum Einsatz im Dentalbereich, beispielsweise in Gemischen und Erzeugnisse zum Beschichten von Zahnoberflächen, nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Phosphaten, Sulfiden, Seleniden und Telluriden von Metallen und deren Mischungen. Besonders bevorzugt sind nanoskalige Teilchen von SiO₂, TiO₂, ZrO₂, ZnO, SnO₂ und Al₂O₃ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

Um eine gute Einbindung der Nanopartikel in die Polymermatrix eines erfindungsgemäßen härtbaren Gemisches oder Erzeugnisses zu erreichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan, wodurch silanisierte Nanopartikel gebildet werden. Als Haftvermittler eignet sich hierbei besonders das Methacryloxypropyltrimethoxysilan.

In einer weiteren bevorzugten Ausgestaltung sind die nanoskaligen Teilchen nicht agglomerierte, organisch oberflächenmodifizierte Nanopartikel mit einer mittleren Partikelgröße kleiner 200 nm, vorzugsweise kleiner 100 nm, besonders bevorzugt kleiner 70 nm, insbesondere im Bereich von 5 bis 60 nm, beispielsweise dispergiert in einem Medium, vorzugsweise in monodisperser Form, wobei diese Nanopartikel wiederum bevorzugt silanisiert sind.

### Bestandteil (b-3) - Photoinitiatoren

Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Phosphonoxide, Acylphosphinoxide, Aryliodoniumsalze, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2, welche im Wege der Verweisung Bestandteil der vorliegenden Anmeldung sind.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Initiatoren sowie Initiatorkombinationen sind in der DE 601 16 142 beschrieben, welche im Wege der Verweisung Bestandteil der vorliegenden Anmeldung sind.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen härtbaren Gemisches bewirken können.

Das Absorptionsmaximum von Campherchinon (CC) liegt bei ca. 470 nm und somit im Bereich des blauen Lichts. Campherchinon (CC) zählt zu den PI₂-Initiatoren und wird regelmäßig zusammen mit einem Coinitiator eingesetzt.

Vorzugsweise enthält eine erfindungsgemäßes härtbares Gemisch die Kombination eines alpha-Diketons und eines aromatischen tertiären Amins, bevorzugt ist die Kombination von Campherchinon (CC) und Ethyl-p-N,N-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in erfindungsgemäßen härtbaren Gemischen wird auf die Druckschriften DE 38 01 511 1 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eigenen sich besonders allein oder in Kombination mit dem System "alpha-Diketon/Amin" als Photopolymerisationsinitiatorsystem in den erfindungsgemäßen Gemischen.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden, welche im Wege der Verweisung Bestandteil der vorliegenden Anmeldung sind.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. 11, Elsevier Applied Science, London, New York 1993 beschrieben, welche im Wege der Verweisung Bestandteil der vorliegenden Anmeldung sind.

### Bestandteil (b-3) - Initiatoren für die chemische Aushärtung

Dem Fachmann sind diverse Initiatoren für eine chemische Aushärtung bekannt. Es sei insoweit exemplarisch auf die EP 1 720 506 verwiesen.

Geeignete Initiatoren für die chemische radikalische Härtung sind unter anderem Peroxide und Amine oder Sulfinsäuren/-säuresalzen wie beispielsweise Dibenzoylperoxid (BPO) in Verbindung mit N,N-bis(hydroxyethyl)paratoluidin (N,N-Bis) oder Natriumsulfinat sowie Barbitursäurederivate wie Benzylphenylbarbitursäure neben löslichen Kupfer- und Tetraalkylammoniumchloridsalzen, Trialkylborane und Azobisisobutyronitril (AIBN).

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023, welche im Wege der Verweisung Bestandteil der vorliegenden Anmeldung sind.

Geeignete Malonylsulfamide sind in der EP 0 059 451 beschrieben welche im Wege der Verweisung Bestandteil der vorliegenden Anmeldung ist. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

### Bestandteil (b-4) - Polymerisationsinhibitoren

Die erfindungsgemäßen härtbaren Gemische enthalten vorzugsweise einen oder mehrere Inhibitoren, auch Stabilisatoren genannt. Diese werden einem härtbaren Gemisch zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität des härtbaren, insbesondere lichthärtbaren, vorzugsweise dentalen, Gemisches.

Gängige Inhibitoren sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Diese Stabilisatoren können auch zur Regelung der Redoxinitiierung eingesetzt werden.

### Bestandteil (b-5) - Lösungsmittel

Vorzugsweise enthält ein erfindungsgemäßes Gemisch ein oder mehrere Lösungsmittel, vorzugsweise in einer Gesamtmenge von 5 bis 65 Gew.-%, bevorzugt in einer Gesamtmenge von 10 bis 50 Gew.-%, jeweils bezogen auf die Gesamtmasse des Gemisches.

Als Lösungsmittel kann ein erfindungsgemäßes Gemisch Wasser enthalten.

Geeignet sind ferner die üblicherweise verwendeten organischen Lösungsmittel, wie beispielsweise Kohlenwasserstoffe, Ketone und Ester wie beispielsweise Toluol, Xylol, Isooctan, Aceton, Butanon, Methylisobutylketon, Ethylacetat, Butylacetat, Tetrahydrofuran, N-Methylpyrrolidon, Dimethylacetamid und Dimethylformamid. Es können auch Alkohole wie Ethanol, Propanole, Butanole, Pentanole, Hexanole, Cyclohexanol, Heptanole, Octanole, Nonanole, Decanole, etc. eingesetzt werden. Ebenfalls geeignet sind cycloaliphatische oder arylaliphatische Alkohole.

In einer bevorzugten Ausgestaltung enthält ein erfindungsgemäßes Gemisch ein organisches Lösungsmittel, vorzugsweise ausgewählt aus der Gruppe bestehend aus mit Wasser mischbaren organischen Lösungsmitteln, vorzugsweise Aceton, Ethanol, n-Propanol und Isopropanol sowie deren Mischungen.

Besonders bevorzugt enthält ein erfindungsgemäßes Gemisch Wasser und zumindest ein mit Wasser mischbares organisches Lösungsmittel, dabei bevorzugt Aceton. Dabei liegt das Verhältnis von Aceton zu Wasser vorzugsweise im Bereich von 1 : 1 bis 10 : 1, bevorzugt im Bereich von 2 : 1 zu 8 : 1, weiter bevorzugt im Bereich von 3 : 1 zu 5 : 1.

### Bestandteil (b-6) - Haftfördernde Additive

Um eine weiter verbesserte Haftung an Zahnschmelz und/oder Dentin zu erreichen, können erfindungsgemäße Verbindungen vorzugsweise mit ein oder mehreren weiteren haftfördernden Additiven kombiniert werden.

Bevorzugt sind daher erfindungsgemäße Gemische, die als Komponente (b-6) ein oder mehrere weitere haftfördernde Additive enthalten. Dabei gelten die als bevorzugt bzw. besonders bevorzugt gekennzeichneten Ausgestaltungen auch hier entsprechend.

Weiter bevorzugt sind erfindungsgemäße Gemische umfassend
(a) eine oder mehrere erfindungsgemäße Verbindungen oder eine erfindungsgemäße Mischung, vorzugsweise in einer der als bevorzugt bzw. besonders bevorzugt gekennzeichneten Ausgestaltungen, und
(b-6) ein oder mehrere haftfördernde Additive, ausgewählt aus der Gruppe bestehend aus polymerisierbaren oder nicht polymerisierbaren Säuren oder Carbonsäureanhydriden, vorzugsweise aus der Gruppe bestehend aus Phosphorsäuren, Phosphonsäuren, Carbonsäuren und deren Salze, Carbonsäureestern und Carbonsäureanhydriden, bevorzugt in einer Menge im Bereich von 0,1 bis 35 Gew.-%, weiter bevorzugt in einer Menge im Bereich von 0,25 bis 25 Gew.-%, insbesondere bevorzugt in einer Menge im Bereich von 0,5 bis 15 Gew.-%, jeweils bezogen auf die Gesamtmasse des Gemisches.

Vorzugsweise sind die ein oder mehreren weiteren haftfördernden Additive der Komponente (b-6) ausgewählt aus der Gruppe bestehend aus 10-(Meth)acryloyloxydecyldihydrogenphosphat (10-MDP), 2-(Meth)acryloyloxyethyl-dihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogen-phosphat, 2-(Meth)acryloyloxynonyldihydrogenphosphat, 11-(Meth)acryloyloxyun-decyldihydrogenphosphat, 20-(Meth)acryloyloxyeicosyldihydrogenphosphat, 1,3-Di(meth)acyloyloxypropyl-2-dihydrogenphosphat, 2-(Meth)acryloyloxyethylphenyl-dihydrogenphosphat, Di(2-(meth)acyloyloxyethyl)pyrophosphat, Di(2-(meth)acyloyloxypropyl)pyrophosphat, Di(2-(meth)acyloyloxybutyl)pyrophosphat, Di(2-(meth)acyloyloxypentyl)pyrophosphat, das Di(2-(meth)acyloyloxyhexyl)pyrophosphat, Di(2-(meth)acyloyloxydecyl)pyrophosphat, Mono-, Di- und/oder Triester der Phosphorsäure, welche durch Umsetzung von Hydroxy-C2-C8-alkylmethacrylat (dabei vorzugsweise Hydroxyethylmethacrylat) oder Glyceryldimethacrylat mit Phosphoroxychlorid erhalten werden, Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat, Methacryloyloxyethylphthalat, Methacryloyloxyethylmaleat, Methacryloyloxyethylsuccinat, 1,3-Glyceroldimethacrylatmaleat und Di-Oxyethoxymethacrylsäureethylendiamintetraessigsäureester.

Dabei wiederum bevorzugte weitere haftfördernde Additive der Komponente (b-6) sind 10-(Meth)acryloyloxydecyldihydrogenphosphat (10-MDP), Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryl-oyloxyethylester (4-MET), Trimellitsäureanhydrid-4-methacryloyloxyethylester (4-META), Pyromellitsäuredimethacrylat, Pyromellitsäureglyceroldimethacrylat.

Im Folgenden wird die Erfindung für Monomere umfassend tricyclische Strukturelemente Q am Beispiel von Tricyclo[5.2.1.0^{2,6}]decan (TCD) - Derivaten näher erläutert.

### 1.) Ausgehend vom Bis(hydroxymethyl)tricyclo[5.2.1.0^{2,6}]decan (TCD-Diol)

Bis(hydroxymethyl)tricyclo[5.2.1.0^{2,6}]decan ist kommerziell erhältlich, beispielsweise als Dicidolgemisch der isomeren Verbindungen 3,8-Bis(hydroxymethyl)tricyclo[5.2.1.0^{2,6}]decan und 4,8-Bis(hydroxymethyl)tricyclo[5.2.1.0^{2,6}]decan sowie 3,9-Bis(hydroxymethyl)tricyclo[5.2.1.0^{2,6}]decan und 4,9-Bis(hydroxymethyl)tricyclo[5.2.1.0^{2,6}]decan.

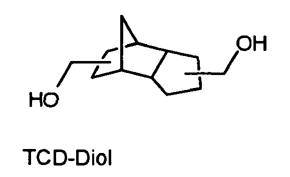

Die Bis(hydroxymethyl)tricyclo[5.2.1.0^{2,6}]decane können, ausgehend von Dicyclopentadien (Tricyclo[5.2.1.0^{2,6}]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.0^{2,6}]decan. Je nach Synthesepfad können gezielt an unterschiedlichen Positionen substituierte Bis(hydroxymethyl)tricyclo[5.2.1.0^{2,6}]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.0^{2,6}]decan herstellbar ist. Die DE 103 52 260 B3 beschreibt dagegen Verfahren zur Herstellung von 3(4), 8(9)-Bis(hydroxymethyl)tricyclo[5.2.1.0^{2,6}]decan. Die Notation der Positionen der Hydroxymethylgruppen 3(4), 8(9) bedeutet 3 oder 4, 8 oder 9.

Die Umsetzung der 3(4), 8(9)-Bis(hydroxymethyl)tricyclo[5.2.1.0^{2,6}]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.0^{2,6}]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

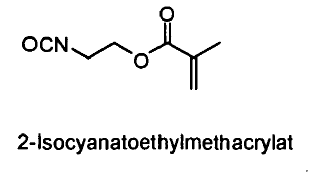

Die Umsetzung von 3(4), 8(9)-Bis(hydroxymethyl)tricyclo[5.2.1.0^{2,6}]decan mit 2-Isocyanatoethylmethacrylat ergibt die Verbindung der Formel (1).

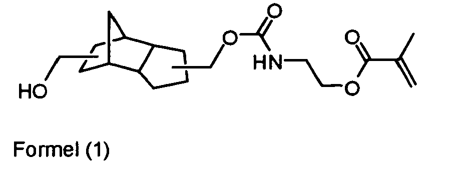

Die Umsetzung von 3(4), 8(9)-Bis(hydroxymethyl)tricyclo[5.2.1.0^{2,6}]decan mit Methacryloylisocyanat ergibt die Verbindung der Formel (2).

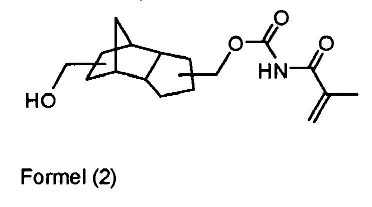

Die jeweilige Umsetzung der Verbindung der Formel (1) bzw. der Formel (2) mit POCl₃ ergibt nach Hydrolyse die erfindungsgemäße Verbindung der Formel (3) bzw. der Formel (4).

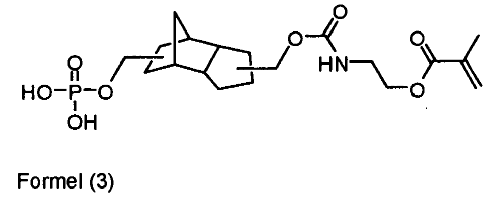

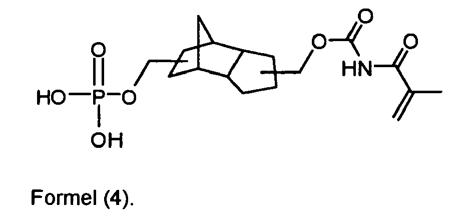

Die Umsetzung der Verbindung der Formel (1) mit Ethylenglycol ergibt die Verbindung der Formel (5), deren anschließende Umsetzung mit POCl₃ ergibt nach Hydrolyse die erfindungsgemäße Verbindung Formel (6).

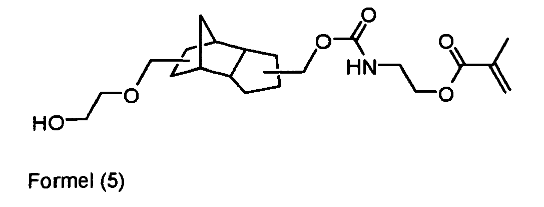

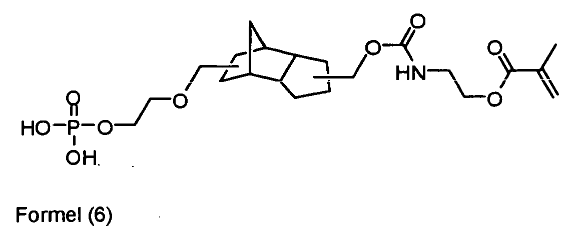

Entsprechende Umsetzungen zu Vergleichszwecken:
Ausgehend von TCD-Diol wurde zu Vergleichszwecken durch Umsetzung mit Methacrylsäurechlorid die nicht erfindungsgemäße Verbindung der Formel (7) erhalten, vgl. zur Synthese auch Beispiel 3. Die weitere Umsetzung von Verbindung (7) mit POCl₃ und anschließender Hydrolyse ergab die nicht erfindungsgemäße Verbindung der Formel (8), vgl. zur Synthese auch Beispiel 4.

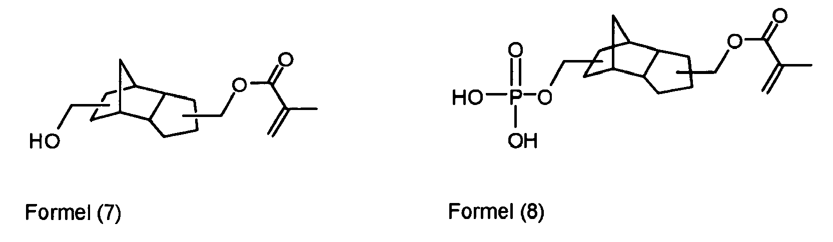

### 2.) Ausgehend von 3(4), 8(9)-Bis(Aminomethyl)tricyclo[5.2.1.0^{2,6}]decan

Das 3(4), 8(9)-Bis(Aminomethyl)tricyclo[5.2.1.0^{2,6}]decan ist an sich bekannt und kann beispielsweise durch Umsetzung der entsprechenden Tosylate mit Ammoniak hergestellt werden.

Reaktion des 3(4), 8(9)-Bis(Aminomethyl)tricyclo[5.2.1.0^{2,6}]decans mit 2-Isocyanatoethylmethacrylat ergibt die Harnstoffverbindung der Formel (9), welche gewissermaßen eine Zwischenprodukt der in EP 0 209 700 A2 beschriebenen Di-Harnstoffverbindung ist.

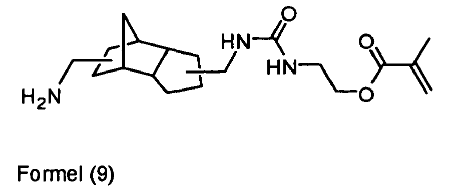

Die Umsetzung von 3(4), 8(9)-Bis(Aminomethyl)tricyclo[5.2.1.0^{2,6}]decan mit Methacryloylisocyanat ergibt die Verbindung der Formel (10).

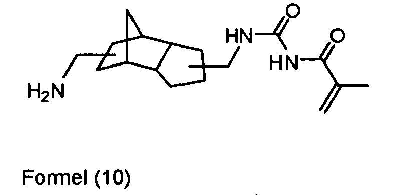

Die jeweilige Umsetzung der Verbindung der Formel (9) bzw. der Formel (10) mit POCl₃ ergibt nach Hydrolyse die erfindungsgemäße Verbindung der Formel (11) bzw. der Formel (12).

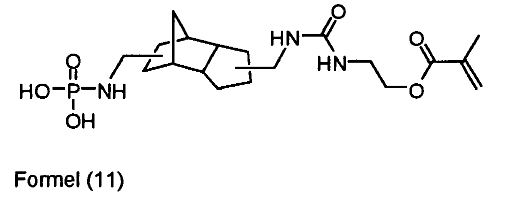

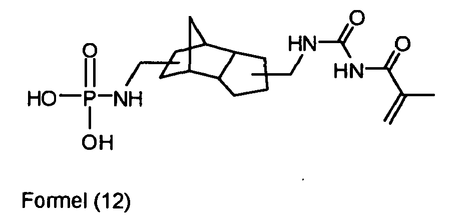

Die Umsetzung der Verbindung der Formel (9) mit Ethylenglycol ergibt die Verbindung der Formel (13), deren anschließende Umsetzung mit POCl₃ ergibt nach Hydrolyse die erfindungsgemäße Verbindung der Formel (14).

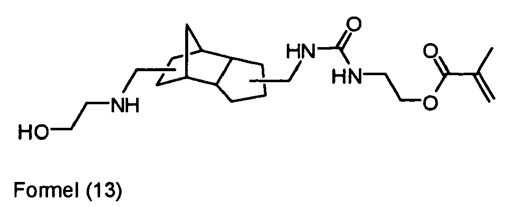

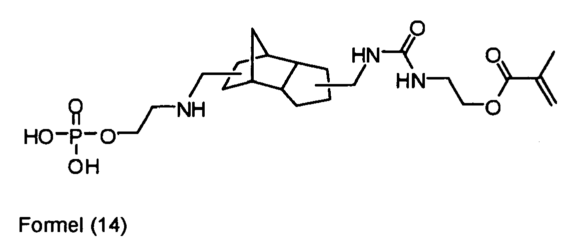

### 3.) Ausgehend von 3(4), 8(9)-Bis(isocyanatomethyl)tricyclo[5.2.1.0^{2,6}]decan

Das 3(4), 8(9)-Bis(isocyanatomethyl)tricyclo[5.2.1.0^{2,6}]decan ist an sich bekannt und zählt zu den herkömmlichen, in industriellen Anwendungen eingesetzten Düsocyanatverbindungen (siehe hierzu die DE 37 03 120 A1 sowie die WO 2009/065873 A2).

Reaktion des 3(4), 8(9)-Bis(isocyanatomethyl)tricyclo[5.2.1.0^{2,6}]decans mit 2-Hydroxyethylmethacrylat (HEMA) ergibt das Urethan der Formel (15).

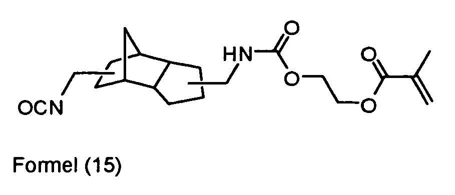

Anstelle von 2-Hydroxyethylmethacrylat (HEMA) können in dieser exemplarisch dargelegten Umsetzungen auch andere Hydroxylverbindungen von (Meth)acrylaten eingesetzt werden, wobei auch Gemische von Acrylaten und Methacrylaten verwendet werden können. Bevorzugt sind daneben auch Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat und Hydroxydodecyl(meth)acrylat, wobei diesbezüglich 2-Hydroxyethylmethacrylat (HEMA) am meisten bevorzugt ist.

Reaktion des 3(4), 8(9)-Bis(isocyanatomethyl)tricyclo[5.2.1.0^{z},⁶]decans mit 2-Methacrylsäure ergibt unter Decarboxylierung das Methacrylamid der Formel (16).

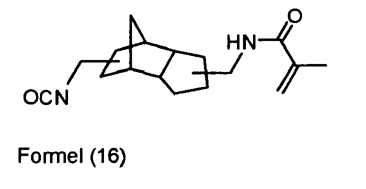

Die Umsetzung der Verbindung der Formel (15) bzw. (16) mit Ethanolamin (entsprechend 2-Aminoethanol) ergibt die nicht erfindungsgemäßen Verbindungen der Formel (17) und (18); deren jeweilige anschließende Umsetzung mit POCl₃ ergibt nach Hydrolyse die erfindungsgemäßen Verbindungen der Formel (19) bzw. der Formel (20).

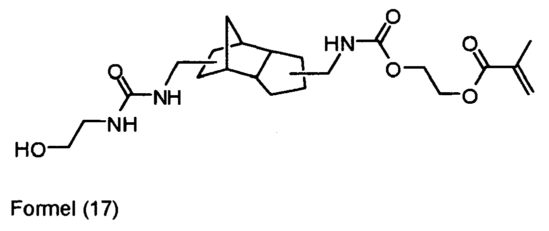

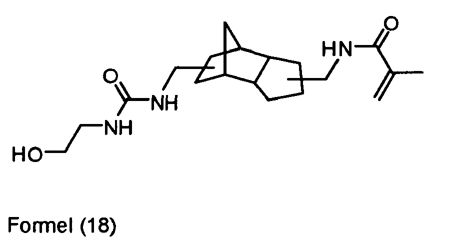

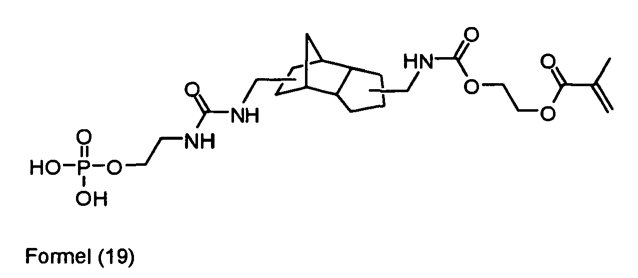

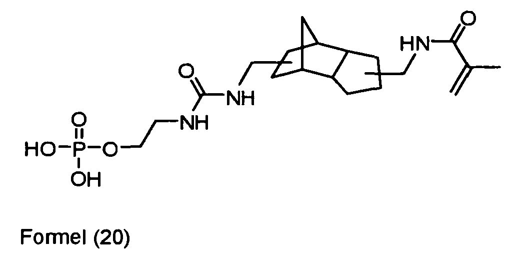

Die Herstellung der Verbindungen der Formel HG-(L)ₓ-Q(YZ)_{b} wird im Allgemeinen in einem inerten Lösungsmittel wie THF (Tetrahydrofuran), Toluol, Xylol, Methylenchlorid oder Acetonitril durchgeführt. Man kann die Umsetzung gegebenenfalls auch lösungsmittelfrei ausführen.

Es gibt zwei Hauptklassen geeigneter Katalysatoren für die Isocyanatadditionsreaktion: zum einen tertiäre Amine (wie Tri-N,N-dimethylaminomethylphenol oder 1,4-Diazabicyclo(2,2,2)octan auch Triethylendiamin genannt), die durch Abstraktion des Hydroxylwasserstoffatoms und Ausbildung von Alkoholatanionen die Alkohole aktivieren und so ihren nukleophilen Angriff auf das Isocyanatkohlenstoffatom beschleunigen, sowie eine Reihe von metallorganischen Verbindungen, die als Lewis Säuren die Elektrophilie des Isocyanatkohlenstoffatoms erhöhen. Bevorzugt eingesetzt werden für die erfindungsgemäßen Umsetzungen Metallsalze höherer Fettsäuren wie Dibutylzinndilaurat, Zinn(II)octoat, etc. oder Verbindungen wie das Eisen(III)acetylacetonat. Ganz bevorzugt ist der Einsatz von Dibutylzinndilaurat.

Der Katalysator wird vorzugsweise in Mengen von 0,01 bis 2 Gew.-%, bevorzugt von 0,08 bis 1 Gew.-%, bezogen auf die Gesamtmenge der Reaktanden eingesetzt.

In einer bevorzugten Ausführungsform kann das Verfahren die Zugabe eines Polymerisationsinhibitors umfassen. Gängige und geeignete Inhibitoren sind beispielsweise Hydrochinonmonomethylether, Hydrochinon, 2,6-Di-tert.-butyl-4-methylphenol. Weitere geeignete Inhibitoren sind in der EP 0 783 880 genannt. Die Zugabe der Inhibitoren erfolgt im Allgemeinen in einer Menge von 0,001 bis 1 Gew.-%, bevorzugt von 0,01 bis 0,5 Gew.-%, bezogen auf die Gesamtmenge der Reaktanden.

Das Verfahren wird bevorzugt unter Ausschluss von Wasser durchgeführt. Bei der Synthese wird dann vorzugsweise eine Planschliffapparatur benutzt, an welcher ein Rührwerk, ein Kühler mit aufgesetztem Trockenrohr, das mit getrocknetem Kieselgur bestückt ist, ein regelbares Thermometer, das die Heizrate des Heizpilzes steuert, und ein Tropftrichter angeschlossen sind. Die Apparatur wird vor der Bestückung mit den Edukten mit Hilfe einer Bunsenbrennerflamme ausgeheizt.

Die Reaktion erfolgt vorzugsweise in einem Temperaturbereich von 0 bis 160°C, bevorzugt im Bereich von 30 bis 140 °C und besonders bevorzugt im Bereich von 60 bis 120 °C. Die Umsetzung wird vorzugsweise bei Normaldruck (1013 mbar) ausgeführt.

Der Fortgang der Reaktion wird mit Hilfe der Änderung der Konzentration an Isocyanatgruppen verfolgt. Die Umsetzung der Isocyanatgruppen kann entweder auf nasschemischem oder spektroskopischem Wege verfolgt werden. Das nasschemische Prinzip der Analyse der Isocyanatgruppen beruht auf der quantitativen Umsetzung des Isocyanats mit einem Überschuss an Dibutylamin und Rücktitration des überschüssigen Amins mit Salzsäure gegen Bromphenolblau bis zum Umschlag von blau nach gelb. Spektroskopisch absorbieren NCO-Gruppen im Wellenlängenbereich von 2275 bis 2250 cm-'. Die Bande zeigt in diesem Bereich eine sehr hohe Intensität, deren Lage auch durch Konjugation nicht beeinflusst wird. Den charakteristischen Wellenlängenbereich der NCO-Bande erkennt man, wenn ein rein qualitatives Spektrum der Isocyanatverbindung in einem geeigneten Lösungsmittel, welches auch für die weiteren Synthesen verwandt werden soll, erstellt wird. Das Lösungsmittel darf keine Absorptionsbanden im Bereich der Wellenlänge aufweisen, der die charakteristischen Absorptionsbanden der NCO-Gruppe anzeigt. Benutzt man beispielsweise Toluol als Lösungsmittel, so kann man als "Fenster", also als den Wellenlängenbereich der charakteristischen Absorptionsbande, das Extinktionsmaximum der NCO-Bande bei 2267 cm -1 wählen.

Die Reaktion wird bis zum vollständigen Verschwinden der Isocyanatbande betrieben.

Die Verbindungen der Formel HG-(L)ₓQ(YZ)_{b} können vorteilhafterweise auch ohne spezielle Verfahren zur Aufreinigung weiter eingesetzt werden.

Zur Herstellung der erfindungsgemäßen Verbindungen wird die Komponente HG-(L)_{X} Q(YZ)_{b} mit POCl₃ oder Phosphor(V)-oxid umgesetzt.

Die Herstellung der erfindungsgemäßen Verbindungen wird in einem inerten Lösungsmittel, vorzugsweise einem Ether wie THF (Tetrahydrofuran), Diethylether, Methyl-tert butylether oder 1,4-Dioxan durchgeführt. Das Lösungsmittel sollte frei von Wasser sein.

In einer bevorzugten Ausführungsform umfasst das Verfahren die Zugabe eines Polymerisationsinhibitors. Gängige und geeignete Inhibitoren sind beispielsweise Hydrochinonmonomethylether, Hydrochinon, 2,6-Di-tert.-butyl-4-methylphenol. Weitere geeignete Inhibitoren sind in der EP 0 783 880 genannt. Die Zugabe der Inhibitoren erfolgt im Allgemeinen in einer Menge von 0,001 bis 1 Gew.-%, bevorzugt von 0,01 bis 0,5 Gew.-%, bezogen auf die Gesamtmenge der Reaktanden.

Das Verfahren wird unter Ausschluss von Wasser durchgeführt. Bei der Synthese wird eine Planschliffapparatur benutzt, an welcher ein Rührwerk, ein Kühler mit aufgesetztem Trockenrohr, das mit getrocknetem Kieselgur bestückt ist, ein Innenthermometer und ein Tropftrichter angeschlossen sind. Die Apparatur wird vor der Bestückung mit den Edukten mit Hilfe einer Bunsenbrennerflamme ausgeheizt.

Zur Erhöhung der Reaktivität kann die Komponente HG-(L)ₓQ(YZ)_{b} in einem ersten Schritt durch Zugabe einer basischen Komponente, wie zum Beispiel einem Alkalihydroxid, einem tertiären organischen Amin oder einem tertiären Alkoholat zunächst deprotoniert werden. Bevorzugt wird hierfür ein tertiäres Amin, besonders bevorzugt Triethylamin oder Pyridin verwendet. Die Deprotonierung wird durch Lösen der Komponente HG-(L)ₓ-Q(YZ)_{b} in dem inerten Lösungsmittel und anschließende Zugabe der basischen Komponente erreicht.

Die Reaktion erfolgt vorzugsweise in einem Temperaturbereich von -90°C bis 20 °C und vorzugsweise im Bereich von -70 °C bis 0 °C. Die Umsetzung wird vorzugsweise bei Normaldruck (1013 mbar) ausgeführt.

Die Reaktionsdauer beträgt vorzugsweise zwischen 0,5 und 24 Stunden. In der Regel kann sie zwischen 0,5 und 3 Stunden abgeschlossen werden.

Die Hydrolyse des Zwischenproduktes aus Umsetzungsschritt ii) des erfindungsgemäßen Herstellungsverfahrens erfolgt durch Zugabe von Wasser. Um im Falle der Umsetzung mit POCl₃ eine Umkehrung der Umsetzung zu verhindern, wird der frei werdende Chlorwasserstoff mit einer geeigneten Base gebunden. Im Allgemeinen wird hierfür ein tertiäres Amin, vorzugsweise Triethylamin oder Pyridin verwendet.

Die erfindungsgemäßen Monomere können einzeln, als Mischungen umfassend zwei oder mehr erfindungsgemäße Monomere sowie in Mischungen mit einem oder mehr herkömmlichen Monomeren sowie sogenannten Vernetzern eingesetzt werden. Durch die Mischung von zwei oder mehr verschiedenen erfindungsgemäßen Monomeren oder von einem, zwei oder mehr erfindungsgemäßen Monomeren mit einem, zwei oder mehr herkömmlichen Monomeren kann beispielsweise die Viskosität dem beabsichtigten Verwendungszweck angepasst werden. So können erfindungsgemäße Monomere beispielsweise mit Comonomeren niedriger Viskosität kombiniert werden.

Die erfindungsgemäßen Monomere können überall eingesetzt werden, wo flüssige, fließfähige Ausgangsmaterialien zu festen Endprodukten ausgehärtet werden. Der Übergang von der flüssigen zur festen Phase wird dabei chemisch, mittels Strahlung oder dual (d.h. sowohl chemisch als auch mittels Strahlung) initiiert. Der Mechanismus der Aushärtung erfolgt radikalisch und/oder ionisch. Einsetzbare Polymerisationsinitiatoren sind somit Photoinitiatoren und thermische Polymerisationskatalysatoren. Dem Fachmann sind radikalische Photoinitiatoren, radikalische Thermoinitiatoren, kationische Photoinitiatoren und kationische Thermoinitiatoren sowie deren Kombinationen bekannt.

Erfindungsgemäße Gemische umfassend die erfindungsgemäßen Monomere können verschiedene, dem Verwendungszweck angepasste Additive, Aktivatoren, Coinitiatoren, Lösungsmittel, Füllstoffe, Stabilisatoren, Pigmente, Reaktivverdünner, Comonomere, Inhibitoren, Molekulargewichtsregler, Fließmittel, Verlaufmittel, Hautverhinderungsmittel, Entschäumer, Antistatika, Weichmacher, Gleitmittel, Netz- und Dispergiermittel, Konservierungsmittel wie beispielsweise Fungizide und/oder Biozide, Modifikatoren zur Einstellung der Rheologie wie Thixotropiermittel und/oder Eindickmittel, Sensibilisatoren, grenzflächenaktiven Substanzen, Sauerstoff- und/oder Radikalfänger, Pigmente, Farbstoffe, Lichtschutzmittel, Mattierungsmittel, Brandschutzmittel, Trennmittel, usw. enthalten.

UV-Absorber, die beispielsweise durch ihre konjugierten Doppelbindungssyteme und aromatischen Ringe befähigt sind, UV Strahlung zu absorbieren, können gegebenenfalls zusätzlich Bestandteil eines erfindungsgemäßen Gemisches bzw. Erzeugnisses sein. Beispiele an UV-Absorbern sind 2-Hydroxy-4-methoxybenzophenon, Salicylsäurephenylester oder 3-(2'-Hydroxy-5'-methylphenyl)-benzotriazol.

Ein erfindungsgemäß bevorzugtes Gemisch oder Erzeugnis umfasst als Additiv ferner eine oder mehrere fluoridabgebende Substanzen, dabei vorzugsweise Natriumfluorid und/oder Aminfluoride.

Ferner können ein oder mehrere Tenside Bestandteil eines erfindungsgemäßen Gemisches oder Erzeugnisses sein.

Die Erfindung betrifft ferner ein Erzeugnis, erhältlich durch Härten einer erfindungsgemä-βen Verbindung, einer erfindungsgemäßen Mischung oder eines erfindungsgemäßen Gemisches.

Bei einem erfindungsgemäßen Erzeugnis handelt es sich vorzugsweise um ein Polymer oder einen Verbundwerkstoff, vorzugsweise ein dentales Polymer oder einen dentalen Verbundwerkstoff.

Die Erfindung betrifft ferner eine erfindungsgemäße Verbindung, eine erfindungsgemäße Mischung, ein erfindungsgemäßes Gemisch oder ein erfindungsgemäßes Erzeugnis als Dentalmaterial bzw. zur Anwendung als Dentalmaterial.

Die Erfindung betrifft ferner die Verwendung einer erfindungsgemäßen Verbindung, einer erfindungsgemäßen Mischung, eines erfindungsgemäßen Gemisches oder eines erfindungsgemäßen Erzeugnisses zur Herstellung eines Dentalmaterials.

Einsetzbar sind die erfindungsgemäßen Verbindungen, Mischungen und Gemische vorzugsweise in oder zur Herstellung von Verbundwerkstoffen und in oder zur Herstellung von Dentalmaterialien (Dentalzusammensetzungen).

Die Erfindung betrifft ferner ein Verfahren zur Herstellung eines Erzeugnisses, vorzugsweise eines dentalen Erzeugnisses, mit folgenden Schritten:
(i) Bereitstellen einer erfindungsgemäßen Verbindung, einer erfindungsgemäßen Mischung oder eines erfindungsgemäßen Gemisches, jeweils vorzugsweise in einer der als bevorzugt oder besonders bevorzugt gekennzeichneten Ausgestaltungen, als erste Komponente,
(ii) gegebenenfalls Herstellen einer Zubereitung durch Vermischen der bereitgestellten ersten Komponente mit einer oder mehreren weiteren Komponenten, vorzugsweise mit einem oder mehreren weiteren dentalen Materialien,
(iii) Härten des oder der Bestandteile aus Schritt (i) oder der Zubereitung gemäß Schritt (ii), wobei das Härten vorzugsweise chemisch und/oder durch Licht induziert und/oder thermisch induziert erfolgt.

In einer bevorzugten Ausgestaltung wird dazu die erste Komponente oder die Zubereitung gemäß Schritt (ii) vor dem Härten in Schritt (iii) aufgetragen auf, eingefügt in und/oder angebracht an die dafür vorgesehene Stelle, vorzugsweise einer Stelle in der Mundhöhle, wobei diese Stelle vorzugsweise zumindest einen oder mehrere Bereiche der Mundhöhle umfasst aus der Gruppe bestehend aus Zahnsubstanz (ein oder mehrere Zähne oder Teile eines Zahns (insbesondere Zahnstumpf, Schmelz, Dentin, Pulpa, Zahnhals, Zahnrand)), Zahnfleisch und/oder einem Bereich unterhalb eines Zahns (insbesondere Wurzel und Wurzelkanal).

Ferner betrifft die Erfindung eine erfindungsgemäße Verbindung, eine erfindungsgemäße Mischung, ein erfindungsgemäßes härtbares Gemisch oder ein erfindungsgemäßes Erzeugnis, jeweils vorzugsweise in einer der als bevorzugt gekennzeichneten Ausgestaltungen,
- in oder zur Herstellung von Haftmitteln, vorzugsweise dentalen Haftmitteln,
- in oder zur Herstellung von Füllungs- und/oder Versiegelungsmaterialien, insbesondere dentalen Füllungsmaterialien, Unterfüllungsmaterialien, Fissurenversiegelungsmaterialien, Wurzelkanalfüll- und/oder - versiegelungsmaterialien,
- in oder zur Herstellung von Lacken, vorzugsweise Glanzlacken und/oder Oberflächenlacken, insbesondere dentalen Glanzlacken und/oder Oberflächenlacken,
- in oder zur Herstellung von fließfähigen Kompositmaterialien (Flow-Materialien), vorzugsweise dentalen Kompositmaterialien,
- in oder zur Herstellung von dentalen provisorischen Restaurationsmaterialien (vorzugsweise provisorischen Inlays, Onlays, Kronen, Brücken, Befestigungsmaterialien) und/oder Stumpfaufbaumaterialien,
- in oder zur Herstellung von Kleb-, Farb-, Anstrichs- oder Beschichtungszusammensetzungen, Vergussmassen, Dichtungsmassen, Spachtelmassen, Laminierharzen, Formmassen, Bindemitteln oder Gießharzen.

Ferner betrifft die Erfindung ein Verfahren zum Behandeln einer Zahnerkrankung, wobei eine oder mehrere erfindungsgemäße Verbindungen, eine erfindungsgemäße Mischung, ein erfindungsgemäßes härtbares Gemisch bzw. ein erfindungsgemäßes dentales Erzeugnis, vorzugsweise in einer der als bevorzugt angegebenen Ausgestaltungen, als dentales Haftmittel, dentales Füllungsmaterial, dentales Unterfüllungsmaterial, fließfähiges Kompositmaterial, Fissurenversiegler, Wurzelkanalfüll- und -versiegelungsmaterial, provisorisches Restaurationsmaterial (vorzugsweise provisorischen Inlays, Onlays, Kronen, Brücken, Befestigungsmaterialien) und/oder als Stumpfaufbaumaterial eingesetzt wird.

### Beispiele

Anhand der folgenden Beispiele wird die Erfindung weiter erläutert. Sofern nicht anders angegeben, beziehen sich alle Angaben auf das Gewicht. Es werden dabei folgende Abkürzungen verwendet:
BHT = 2,6-Di-tert.butyl-4-methylphenol
UDMA = Urethandimethacrylat (7,7,9-Trimethyl-4,13-dioxo-5,12-diazahexadecan-1,16-dioxydimethacrylat)
4-META = Trimellitsäureanhydrid-4-methacryloyloxyethylester
CC = Campherchinon
DABE = Ethyl-p-N,N-dimethylaminobenzoat
Bis-GMA = Bisphenol-A-Glycidyl-Methacrylat
TEDMA = Triethylenglykoldimethacrylat
TCD-Monomer = Bis(methacryloyloxymethyl)tricyclo[5.2.1.0^{2,6}]decan

Für die im Folgenden eingesetzte Katalysatorlösung wurden 0,50 g Dibutylzinn(11)dilaurat in 9,50 g Toluol gelöst.

### Beispiel 1: Synthese der Verbindung der Formel (1) (nicht erfindungsgemäß):

3,80 g (19,36 mmol) 3(4), 8(9)-Bis(hydroxymethyl)tricyclo[5.2.1.0^{2,6}]decan wurden in 15 mL Tetrahydrofuran gelöst und mit 0,04 g BHT und 0,105 g (ca. 0,1 mol%) der Katalysatorlösung versetzt. Unter Rühren wurden 3,00 g (19,34 mmol) 2-Isocyanatoethylmethacrylat, gelöst in 10 mL THF, zugetropft. Nach beendeter Zugabe wurde der Tropftrichter durch einen Rückflusskühler ausgetauscht und das Reaktionsgemisch auf 60 °C erwärmt und der Fortschritt der Reaktion mittels IR-Spektroskopie verfolgt. Nach 24 Stunden konnte keine Isocyanatbande mehr detektiert werden. Das Lösungsmittel wurde am Rotationsverdampfer entfernt. Das Urethan der Formel (1) wurde in einer Ausbeute von 6,66 g (18,95 mmol, entsprechend 98% der Theorie) erhalten.

### Beispiel 2: Synthese der Verbindung der Formel (3):

2,56 g (16,69 mmol) POCl₃ wurden in 20 mL Methyl-tert-butylether gelöst und auf -78 °C abgekühlt. Unter starkem Rühren wurde eine Mischung aus 5,10 g (14,51 mmol) der Verbindung der Formel (1) aus Beispiel 1 und 1,69 g (16,69 mmol) Triethylamin in 10 mL Methyl-tert-butylether zugetropft. Nach der Zugabe wurde eine Stunde weitergerührt und das Reaktionsgemisch dann bei 0 °C mit 0,91 g (50,79 mmol) Wasser und 3,67 g (36,28 mmol) Triethylamin versetzt. Nachdem eine weitere Stunde bei Raumtemperatur gerührt wurde, wurde das Reaktionsgemisch mit 1%iger Salzsäure und danach mit Wasser aufgearbeitet. Die organische Phase wurde über Magnesiumsulfat getrocknet und das Lösungsmittel anschließend am Rotationsverdampfer entfernt. Es verblieben 3,85 g (8,90 mmol, entsprechend 61 % der Theorie) der Verbindung (3).

### Beispiel 3: Synthese der Verbindung der Formel (7) (nicht erfindungsgemäß):

3,80 g (19,36 mmol) 3(4), 8(9)-Bis(hydroxymethyl)tricyclo[5.2.1.0^{2,6}]decan und 1,70 mL (1,66 g, 21,0 mmol) Pyridin wurden in 50 mL Chloroform gelöst und unter Eiskühlung tropfenweise mit 1,89 mL (2,02 g, 19,36 mmol) Methacrylsäurechlorid, gelöst in 10 mL Chloroform, versetzt. Nach beendeter Zugabe wurde über Nacht bei Raumtemperatur gerührt. Am nächsten Tag wurde die Reaktionsmischung mit 150 mL 1N Salzsäure versetzt in einen Scheidetrichter überführt und die Phasen getrennt. Anschließend wurde noch je zweimal mit gesättigter NaHCO₃- und NaCl-Lösung ausgeschüttelt und abschließend mit Wasser gewaschen. Die organische Phase wurde über MgS04 getrocknet und das Lösungsmittel nach Zusatz von 0,04 g BHT am Rotationsverdampfer entfernt. Der Methacrylsäureester der Formel (7) wurde in einer Ausbeute von 4,93 g (18,78 mmol, entsprechend 97% der Theorie) erhalten.

### Beispiel 4: Synthese der Verbindung der Formel (8) (nicht erfindungsgemäß, vgl, JP 2007091642A):

2,56 g (16,69 mmol) POCl₃ wurden in 20 mL Methyl-*tert*-butylether gelöst und auf -78 °C abgekühlt. Unter starkem Rühren wurde eine Mischung aus 3,81 g (14,51 mmol) der Verbindung der Formel (7) aus Beispiel 3 und 1,69 g (16,69 mmol) Triethylamin in 10 mL Methyl-tert-butylether zugetropft. Nach der Zugabe wurde eine Stunde weitergerührt und das Reaktionsgemisch dann bei 0 °C mit 0,91 g (50,79 mmol) Wasser und 3,67 g (36,28 mmol) Triethylamin versetzt. Nachdem eine weitere Stunde bei Raumtemperatur gerührt wurde, wurde das Reaktionsgemisch mit 1%iger Salzsäure und danach mit Wasser aufgearbeitet. Die organische Phase wurde über Magnesiumsulfat getrocknet und das Lösungsmittel anschließend am Rotationsverdampfer entfernt. Es verblieben 3,85 g (10,45 mmol, entsprechend 72% der Theorie) der Verbindung (8).

### Beispiel 5: Synthese der Verbindung der Formel (21) (nicht erfindungsgemäß

Das Haftmonomer (21) wurde gemäß der in der US 2006/0246017A1 beschriebenen Synthesesequenz des Beispiels 2 unter Austausch des dort verwendeten 1,10-Decandiols durch 3(4), 8(9)-Bis(hydroxymethyl)tricyclo[5.2.1.0^{2,6}]decan dargestellt.

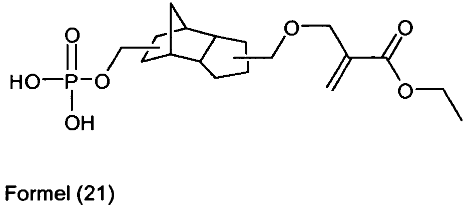

### Dentale Zusammensetzungen:

Es wurden die folgenden Zusammensetzungen hergestellt. Dabei sind die Zusammensetzungen A, B, F, G und H Vergleichsbeispiele, die Zusammensetzungen C, D und E sind erfindungsgemäße Gemische.

4-META ist ein für seine guten haftvermittelnden Eigenschaften bekanntes und in vielen Bereichen eingesetztes haftfördemdes Additiv. Es wurde aufgrund seiner erfolgreichen und verbreiteten Verwendung im Dentalbereich als Referenz herangezogen.

Die Zusammensetzungen der Beispiele A - H (jeweils in Gewichtsteilen) sowie die Ergebnisse der Haftwert-Messungen sind in den folgenden Tabellen aufgelistet. Diese Zusammensetzungen wurden nach dem Non-Etch Verfahren und ohne Primer verarbeitet.

| | **A** | **B** | **C** | **D** | **E** | **F** | **G** | **H** |
|---|---|---|---|---|---|---|---|---|
| HEMA | 15,30 | 15,30 | 15,30 | 15,30 | 5,99 | 5,99 | 15,30 | 15,30 |
| UDMA | 7,65 | 7,65 | 7,65 | 7,65 | 2,99 | 2,99 | 7,65 | 7,65 |
| Bis-GMA | 7,65 | 0,00 | 7,65 | 0,00 | 2,99 | 2,99 | 7,65 | 7,65 |
| TCD-Monomer | 0,00 | 7,65 | 0,00 | 7,65 | 0,00 | 0,00 | 0,00 | 0,00 |
| 4-META | 30,61 | 30,61 | 0,00 | 0,00 | 0,00 | 12,02 | 0,00 | 0,00 |
| Haftmonomer der Formel (3) | 0,00 | 0,00 | 30,61 | 30,61 | 12,02 | 0,00 | 0,00 | 0,00 |
| Haftmonomer der Formel (8) (nicht erfindungsgemäß) | 0,00 | 0,00 | 0,00 | 0,00 | 0,00 | 0,00 | 30,61 | 0,00 |
| Haftmonomer der Formel (21) (nicht erfindungsgemäß) | 0,00 | 0,00 | 0,00 | 0,00 | 0,00 | 0,00 | 0,00 | 30,61 |
| DABE | 0,28 | 0,28 | 0,28 | 0,28 | 0,11 | 0,11 | 0,28 | 0,28 |
| BHT | 0,06 | 0,06 | 0,06 | 0,06 | 0,02 | 0,02 | 0,06 | 0,06 |
| CC | 0,18 | 0,18 | 0,18 | 0,18 | 0,07 | 0,07 | 0,18 | 0,18 |
| Wasser | 7,65 | 7,65 | 7,65 | 7,65 | 2,99 | 2,99 | 7,65 | 7,65 |
| Aceton | 30,61 | 30,61 | 30,61 | 30,61 | 11,97 | 11,97 | 30,61 | 30,61 |
| Glaskeramik (d₅₀ = 1,5 µm) | 0,00 | 0,00 | 0,00 | 0,00 | 39,91 | 39,91 | 0,00 | 0,00 |
| Glaskeramik (d₅₀ = 0,7 µm) | 0,00 | 0,00 | 0,00 | 0,00 | 18,75 | 18,75 | 0,00 | 0,00 |
| pyrogene Kieselsäure | 0,00 | 0,00 | 0,00 | 0,00 | 2,16 | 2,16 | 0,00 | 0,00 |

### Haftwerte an Dentin

Es wurden folgende Haftwerte am Dentin bestimmt.

Die angegebenen Haftwerte sind Mittelwerte aus mindestens 5 Einzelmessungen.

| | **A** | **B** | **C** | **D** | **E** | **F** | **G** | **H** |
|---|---|---|---|---|---|---|---|---|
| Haftwerte in MPa: | 9 | 10 | 15 | 19 | 15 | 4 | 11 | 8 |

Überraschenderweise sind die Haftwerte der erfindungsgemäßen Zusammensetzung C besser als die Haftwerte der Zusammensetzungen G und H, bei der Monomere eingesetzt wurden, die sich an Monomeren aus dem Stand der Technik orientieren.

### Messverfahren zur Bestimmung der Haftwerte:

Zur Bestimmung der Haftungseigenschaften der dentalen Zusammensetzungen A bis H wurden die Haftwerte an extrahierten Rinderzähnen ermittelt. Die Dentinprobenkörper wurden direkt vor ihrer Verwendung aus einer isotonischen Kochsalzlösung entnommen, mit Wasser gespült und unverzüglich mit der jeweils zu untersuchenden Zusammensetzung behandelt. Die Haftwerte sind Mittelwerte aus mindestens 5 Messungen.

### Für die Beispiele A bis D, G und H:

Um eine frische Präparation zu simulieren wurden die freiliegenden Dentinflächen auf feinem Schmirgelpapier (1000er Körnung) feucht beschliffen. Die resultierende Fläche sollte plan sein und nicht austrocknen. Direkt vor der Applikation wurde überschüssige Feuchtigkeit verblasen und die jeweils zu untersuchende Zusammensetzung mittels eines Schwämmchens oder eines Pinsels drei Mal aufgetragen und 10 s einmassiert.

Die jeweilige Zusammensetzung wurde dann 10 s mit einer Blaulichtquelle (Celalux 2, VOCO GmbH Cuxhaven) lichtgehärtet. Danach wurde auf der gehärteten Fläche ein Silikonring mit einem Innendurchmesser von 5 mm aufgesetzt ohne die bestehende Inhibitionsschicht der jeweiligen Zusammensetzung zu verwischen.

In die Öffnung des Silikonringes wurde dann ein lichthärtendes Füllungskomposit auf Methacrylatbasis (Grandio A1, Charge 1019293, VOCO GmbH Cuxhaven) aufgetragen und 40 s lichtgehärtet (zylinderförmige Prüfkörper (3 mm (Höhe) x 5 mm (Durchmesser)). Die fertigen Proben wurden im Probenschrank bei 37°C und 100% relativer Luftfeuchte gelagert und nach 24h wird die Scherhaftung mit Hilfe einer Universalprüfmaschine (1 mm/min) bestimmt. Nach der Messung wurden die exakten Dimensionen des Prüfkörpers für die Berechnung der Haftung (angegeben in MPa) mit einer Messschraube bestimmt.

### Für die Beispiele E und F (Haftzemente):

Für die Untersuchungen zu den Zusammensetzungen E und F (Haftzemente) wurde der Probekörperwie oben beschrieben vorbereitet. Der jeweilige Zement wurde dann auf die angeschliffene Fläche in geschlossener Schicht aufgetragen und 20 s in die Zahnsubstanz eingearbeitet. Dann wurde der jeweilige Zement mit dem Luftbläser gleichmäßig verteilt und 10 s lichtgehärtet (Celalux 2, VOCO GmbH Cuxhaven). Hierbei ist darauf zu achten, dass sich keine Ansammlungen bilden ("Pooling") und die Oberfläche des Probenkörpers gleichmäßig benetzt ist. Nach der Härtung wurde ein Silikonring mit einem Innendurchmesser von 5 mm aufgesetzt ohne die Inhibitionsschicht innerhalb des Ringes zu beschädigen und ein Füllungskomposit (Grandio A1, Charge 1019293, VOCO GmbH Cuxhaven) eingebracht. Nach 40 s Lichthärtung wurde die Probe im 37°C warmen Wasserdampfbad für 24 h gelagert und dann die Scherhaftung wie oben beschrieben ermittelt.

Die Beispiele C bis E wurden unter Austausch des dort jeweils eingesetzten erfindungsgemäßen Haftmonomers der Formel (3) gegen entsprechende erfindungsgemäße Verbindungen mit einem Bicyclo[2.2.1]heptan- bzw. Tricydo[3.3.1.1^{3,7}]decan-Gerüst durchgeführt. Die bestimmten Parameterwerte sind ähnlich zu denen aus den Beispielen C bis E.

## Patentansprüche

1. Verbindung der Struktur (HO)_{w}-P(=O)-[G-(L)ₓ-Q(YZ)_{b}]_{y},
wobei hier und nachfolgend gilt:
- jedes Q bedeutet unabhängig von etwaigen weiteren Gruppen Q ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei Q keinen weiteren Substituenten trägt oder einen oder mehrere Substituenten ausgewählt aus der Gruppe bestehend aus Alkylgruppen, Alkoxygruppen, Halogenatome und Trifluormethylgruppen,
- der Index b ist eine natürliche Zahl ausgewählt aus der Gruppe der natürlichen Zahlen 1, 2 und 3,
- jedes Z bedeutet ein Strukturelement, das unabhängig von etwaigen weiteren Strukturelementen Z ausgewählt ist aus der Gruppe bestehend aus
-(C=O)-CH=CH₂, -(C=O)-C(CH₃)=CH₂ und -CH=CH₂,
- jedes Y bedeutet ein Strukturelement, welches - unabhängig von etwaigen weiteren Strukturelementen Y - ein oder mehrere N-Atome enthält und das polyalicyclische Strukturelement Q mit Z so verbindet, dass in der Q mit Z verbindenden Kette von Atomen ein oder mehrere N-Atome enthalten sind,
- jedes Strukturelement G bedeutet - unabhängig von etwaigen weiteren Strukturelementen G - entweder O oder NH,
- jedes L bedeutet ein Strukturelement, welches - unabhängig von etwaigen weiteren Strukturelementen L - die Gruppe G mit dem polyalicyclischen Strukturelement Q verbindet,
- der Index w ist ausgewählt aus der Gruppe der natürlichen Zahlen 0, 1 und 2,
- jeder Index x bedeutet unabhängig von etwaigen weiteren Indizes x entweder 0 oder 1,
- der Index y ist ausgewählt aus der Gruppe der natürlichen Zahlen 1, 2 und 3,
wobei die Summe von w + y = 3 ist.

2. Verbindung nach Anspruch 1, wobei die Verbindung die folgende Struktur aufweist
(HO)_{w}-P(=O)-[G-(CH₂)ₖ(A-C(=O))ᵣ(E)ⱼ-(CH₂)ₙ-Q(YZ)_{b}]_{y},
wobei gilt:
- jeder Index k ist eine natürliche Zahl, die unabhängig von etwaigen weiteren Indizes k ausgewählt ist aus der Gruppe der natürlichen Zahlen 0 bis 12, vorzugsweise aus der Gruppe der natürlichen Zahlen 0 bis 8,
- jedes Strukturelement A bedeutet - unabhängig von etwaigen weiteren Strukturelementen A - entweder O oder NH,
- jedes Strukturelement E bedeutet - unabhängig von etwaigen weiteren Strukturelementen E - entweder O oder NH,
- jeder Index r bedeutet unabhängig von etwaigen weiteren Indizes r entweder 0 oder 1,
- jeder Index j bedeutet unabhängig von etwaigen weiteren Indizes j entweder 0 oder 1,
- jeder Index n bedeutet unabhängig von etwaigen weiteren Indizes n entweder 0 oder 1,
wobei wenn k = 0 ist, der Index j = 0 und der Index r = 0 bedeutet.

3. Verbindung nach Anspruch 1 oder 2, umfassend eine, zwei oder mehr funktionelle Gruppen ausgewählt aus der Gruppe bestehend aus Ester, Urethan, N-Acylurethan, Harnstoff, *N*-Acylharnstoff und Amid, wobei die Amidfunktion nicht unmittelbar mit einem N-Atom, einem O-Atom oder einer Carbonylgruppe verknüpft ist.

4. Verbindung nach einem der vorangehenden Ansprüche, **dadurch gekennzeichnet, dass** die Gruppierung YZ eine funktionelle Gruppe umfasst ausgewählt aus der Gruppe bestehend aus Ester, Urethan, N-Acylurethan, Harnstoff, N-Acylhamstoff und Amid, wobei die Amidfunktion nicht unmittelbar mit einem N-Atom, einem O-Atom oder einer Carbonylgruppe verknüpft ist.

5. Verbindung nach einem der vorangehenden Ansprüche, **dadurch gekennzeichnet, dass** die Gruppierung YZ eine funktionelle Gruppe umfasst ausgewählt aus der Gruppe bestehend aus Urethan, N-Acylurethan, Harnstoff, N-Acylhamstoff und Methacrylamid, wobei das bzw. zumindest eines der N-Atome dieser funktionellen Gruppe sich in der Q mit Z verbindenden Kette von Atomen befindet.

6. Verbindung nach einem der vorangehenden Ansprüche, **dadurch gekennzeichnet, dass** die Gruppierung YZ eine Struktur aufweist ausgewählt aus der Gruppe bestehend aus
(-CH₂)ₙ-O-C(=O)-NH-Z,
(-CH₂)ₙ-O-C(=O)-NH-(CH₂)ₘ-O-Z,
(-CH₂)ₙ-NH-C(=O)-NH-Z,
(-CH₂)ₙ-NH-C(=O)-NH-(CH₂)ₘ-O-Z,
(-CH₂)ₙ-NH-Z,
und
(-CH₂)ₙ-NH-C(=O)-O-(CH₂)ₘ-O-Z,
wobei Z und n die oben angegebene Bedeutung haben und wobei gilt:
- jeder Index m ist eine natürliche Zahl, die unabhängig von etwaigen weiteren Indizes m ausgewählt ist aus der Gruppe der natürlichen Zahlen 1 bis 12, vorzugsweise aus der Gruppe der natürlichen Zahlen 2 bis 8.

7. Verbindung nach einem der vorangehenden Ansprüche, wobei das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet.

8. Verfahren zur Herstellung einer Verbindung der Struktur (HO)_{w}-P(=O)-[G-(L)ₓ-Q(YZ)_{b}]_{y} nach einem der vorangehenden Ansprüche, oder einer Mischung umfassend zumindest eine Verbindung der Struktur (HO)_{w}-P(=O)-[G-(L)ₓ-Q(YZ)_{b}]_{y} nach einem der vorangehenden Ansprüche, mit folgenden Schritten:
(i) Bereitstellen einer Verbindung HG-(L)ₖ-Q(YZ)_{b},
(ii) Umsetzung der Verbindung aus Schritt (i) mit POCl₃ oder Phosphor(V)-oxid, vorzugsweise in Gegenwart eines Amins, bevorzugt eines tertiären Amins,
(iii) Hydrolyse des in Schritt (ii) gebildeten Umsetzungsproduktes,
wobei G, L, Q, Y, Z, w, x, b und y jeweils die oben genannte Bedeutung haben, und
wobei die molare Menge der Verbindung aus Schritt (i) pro Atomäquivalent Phosphor im Bereich von 0,5 bis 6 liegt, vorzugsweise im Bereich von 0,6 bis 5, bevorzugt im Bereich von 0,7 bis 4, weiter bevorzugt im Bereich von 0,8 bis 3,5.

9. Mischung umfassend eine oder zwei oder mehr unterschiedliche Verbindungen nach einem der Ansprüche 1 bis 7, vorzugsweise herstellbar nach einem Verfahren gemäß Anspruch 8.

10. Härtbares Gemisch, umfassend
(a) eine oder mehrere Verbindungen nach einem der Ansprüche 1 bis 7 oder eine Mischung nach Anspruch 9,
und
(b) einen oder mehrere weitere Bestandteile gewählt aus der Gruppe bestehend aus
(b-1) von Bestandteil (a) verschiedene Monomere, welche mit Bestandteil (a) co-polymerisierbar sind, vorzugsweise lichtpolymerisierbare Monomere,
(b-2) einen oder mehrere Füllstoffe, vorzugsweise einen oder mehrere nanoskalige Füllstoffe,
(b-3) Photoinitiatoren und Initiatoren für die chemische Aushärtung,
(b-4) Polymerisationsinhibitoren,
(b-5) Lösungsmittel,
und
(b-6) von Bestandteil (a) verschiedene haftfördernde Additive.

11. Gemisch nach Anspruch 10, wobei Bestandteil (b-1) besteht aus oder umfasst
(b-1 a) ein oder mehrere (Meth)acrylat-Monomere, vorzugsweise ausgewählt aus der Gruppe bestehend aus 2-Hydroxyethylmethacrylat (HEMA), Bisphenol-A-glycidyl-methacrylat (Bis-GMA), 7,7,9-Trimethyl-4,13-dioxo-5,12-diazahexadecan-1,16-dioxydimethacrylat (UDMA), Triethylenglycoldimethacrylat (TEDMA), Tetraethylenglycoldimethacrylat, Polyethylenglykoldimethacrylat, Trimethylolpropantrimethacrylat (TMPTMA), Dodecandioldimethacrylat (DODMA), Glycerindi(meth)acrylat, 1,6-Hexandioldimethacrylat (HEDMA), ethoxyliertes Bisphenol-A-dimethacrylat, Pentaerythritoldimethacrylat, Pentaerythritol-tri(meth)acrylat und Dipentaerythritolpenta(meth)acrylat,
und/oder
(b-1 b) ein oder mehrere Monomere ausgewählt aus der Gruppe bestehend aus Verbindungen der Struktur Q^{a}(MXₑ)ₕ, wobei gilt:
- Q^{a} ist ein polyalicyclisches Strukturelement und hat - unabhängig von der Bedeutung des Strukturelements Q in Verbindungen des Bestandteils (a) - die oben für Q angegebenen Bedeutung,
- h ist eine natürliche Zahl ausgewählt aus der Gruppe der natürlichen Zahlen 1, 2, 3 und 4,
- jedes X bedeutet ein Strukturelement, das - unabhängig von etwaigen weiteren Strukturelementen X - ausgewählt ist aus der Gruppe bestehend aus
-O-(C=O)-CH=CH₂, -O-(C=O)-C(CH₃)=CH₂,
-(C=O)-CH=CH₂, -(C=O)-C(CH₃)=CH₂ und -O-CH=CH₂,
- jeder Index e ist eine natürliche Zahl, die unabhängig von etwaigen weiteren Indizes e, ausgewählt ist aus der Gruppe der natürlichen Zahlen 1, 2, 3 und 4,
- jedes M bedeutet ein Strukturelement, welches - unabhängig von etwaigen weiteren Strukturelementen M - in der Struktur Q^{a}(MXₑ)ₕ das polyalicyclische Strukturelement Q^{a} mit e Strukturelementen X verbindet.

12. Erzeugnis, erhältlich durch Härten einer Verbindung nach einem der Ansprüche 1 bis 7, einer Mischung nach Anspruch 9 oder eines Gemisches nach einem der Ansprüche 10 und 11.

13. Verbindung nach einem der Ansprüche 1 bis 7, Mischung nach Anspruch 9, Gemisch nach einem der Ansprüche 10 und 11 oder Erzeugnis nach Anspruch 12 als Dentalmaterial oder zur Anwendung als Dentalmaterial.

14. Verwendung einer Verbindung nach einem der Ansprüche 1 bis 7, einer Mischung nach Anspruch 9, eines Gemisches nach einem der Ansprüche 10 und 11 oder eines Erzeugnisses nach Anspruch 12 zur Herstellung eines Dentalmaterials.

15. Verfahren zur Herstellung eines Erzeugnisses, vorzugsweise eines dentalen Erzeugnisses, mit folgenden Schritten:
(i) Bereitstellen einer Verbindung nach einem der Ansprüche 1 bis 7, einer Mischung nach Anspruch 9 oder eines Gemisches nach einem der Ansprüche 10 und 11 als erste Komponente,
(ii) gegebenenfalls Herstellen einer Zubereitung durch Vermischen der bereitgestellten ersten Komponente mit einer oder mehreren weiteren Komponenten, vorzugsweise mit einem oder mehreren weiteren dentalen Materialien,
(iii) Härten des oder der Bestandteile aus Schritt (i) oder der Zubereitung gemäß Schritt (ii), wobei das Härten vorzugsweise chemisch und/oder durch Licht induziert und/oder thermisch induziert erfolgt.

## Claims

1. A compound of the structure (HO)_{w}-P(=O)-[G-(L)ₓ-Q(YZ)_{b}]_{y},
wherein here and below the following applies:
- each Q, Independently of any further groups Q, represents a saturated or olefinically unsaturated polyalicyclic structure element selected from the group consisting of bicyclic, tricyclic, tetracyclic, pentacyclic and hexacyclic hydrocarbon residues, wherein Q carries no further substituent or one or more substituents selected from the group consisting of alkyl groups, alkoxy groups, halogen atoms and trifluoromethyl groups,
- the index b is a natural number selected from the group of natural numbers 1, 2 and 3,
- each Z represents a structure element which, independently of any further structure elements Z, is selected from the group consisting of
-(C=O)-CH=CH₂, -(C=O)-C(CH₃)=CH₂ and -CH=CH₂,
- each Y represents a structure element, which - independently of any further structure elements Y - contains one or more N atoms and binds the polyallcyclic structure element Q with Z in such a way that one or more N atoms are contained in the chain of atoms binding Q with Z,
- each structure element G - independently of any further structure elements G - represents either O or NH,
- each L represents a structure element which - independently of any further structure elements L - binds the group G with the polyalicyclic structure element Q,
- the index w is selected from the group of natural numbers 0, 1 and 2,
- each index x, independently of any further indices x, represents either 0 or 1,
- the index y is selected from the group of natural numbers 1, 2 and 3,
wherein the sum of w + y = 3.

2. The compound according to claim 1, wherein the compound has the following structure
(HO)_{w}-P(=O)-[G-(CH₂)ₖ-(A-C(=O))ᵣ(E)ⱼ-(CH₂)ₙ-Q(YZ)_{b}]_{y},
wherein the following applies:
- each index k is a natural number which, independently of any further indices k, is selected from the group of natural numbers 0 to 12 and preferably from the group of natural numbers 0 to 8,
- each structure element A - independently of any further structure elements A - represents either O or NH,
- each structure element E - independently of any further structure elements E - represents either O or NH,
- each index r, independently of any further indices r, represents either 0 or 1,
- each index j, independently of any further indices j, represents either 0 or 1,
- each index n, independently of any further indices n, represents either 0 or 1,
wherein if k = 0, the index j represents 0 and the index r represents 0.

3. The compound according to claim 1 or 2, comprising one, two or more functional groups selected from the group consisting of ester, urethane, *N*-acyl urethane, urea, *N*-acyl urea and amide, wherein the amide function is not directly linked with an N atom, an 0 atom or a carbonyl group.

4. The compound according to one of the preceding claims, **characterised in that** the grouping YZ comprises a functional group selected from the group consisting of ester, urethane, *N*-acyl urethane, urea, *N*-acyl urea and amide, wherein the amide function is not directly linked with an N atom, an O atom or a carbonyl group.

5. The compound according to one of the preceding claims, **characterised in that** the grouping YZ comprises a functional group selected from the group consisting of urethane, *N*-acyl urethane, urea, *N*-acyl urea and methacrylamide, wherein the N atom or at least one of the N atoms of this functional group is located in the chain of atoms binding Q with Z.

6. The compound according to one of the preceding claims, **characterised in that** the grouping YZ has a structure selected from the group consisting of
(-CH₂)ₙ-O-C(=O)-NH-Z,
(-CH₂)ₙ-O-C(=O)-NH-(CH₂)ₘ-O-Z,
(-CH₂)ₙ-NM-C(=O)-NH-Z,
(-CH₂)ₙ-NH-C(=O)-NH-(CH₂)ₘ-O-Z,
(-CH₂)ₙ-NH-Z,
and
(-CH₂)ₙ-NH-C(=O)-O-(CH₂)ₘ-O-Z,
wherein Z and n have the meaning given above and wherein the following applies:
- each index m is a natural number which, independently of any further indices m, is selected from the group of natural numbers 1 to 12 and preferably from the group of natural numbers 2 to 8.

7. The compound according to one of the preceding claims, wherein the structure element Q represents a tricyclo[5.2.1.0^{2,6}]decane residue, a tricyclo[5.2.1.0^{2,6}]dec-3-ene residue, a tricyclo[3.3.1.1^{3,7}]decane residue or a bicyclo[2.2.1]heptane residue.

8. A method for preparing a compound of the structure (HO)_{w}-P(=O)-[G-(L)ₓ-Q(YZ)_{b}]_{y} according to one of the preceding claims or a mixture comprising at least one compound of the structure (HO)_{w}-P(=O)-[G-(L)ₓ-Q(YZ)_{b}]_{y} according to one of the preceding claims, with the following steps:
(i) providing a compound HG-(L)ₓ-Q(YZ)_{b},
(ii) reacting the compound from step (i) with POCl₃ or phosphorus(V) oxide, preferably in the presence of an amine, preferably a tertiary amine,
(iii) hydrolysis of the reaction product formed in step (ii),
wherein G, L, Q, Y, Z, w, x, b and y each have the above meanings, and
wherein the molar quantity of the compound from step (i) per atom equivalent of phosphorus is in the range of 0.5 to 6, for preference in the range of 0.6 to 5, preferably in the range of 0.7 to 4 and more preferably in the range of 0.8 to 3.5.

9. A mixture comprising one or two or more different compounds according to one of claims 1 to 7, which are preferably preparable by a method according to claim 8.

10. A curable blend comprising
(a) one or more compounds according to one of claims 1 to 7 or a mixture according to claim 9,
and
(b) one or more further constituents selected from the group consisting of
(b-1) monomers differing from constituent (a), which are copolymerisable with constituent (a), preferably light-polymerisable monomers,
(b-2) one or more fillers, preferably one or more nanoscale fillers,
(b-3) photoinitiators and initiators for chemical curing,
(b-4) polymerisation inhibitors,
(b-5) solvents
and
(b-6) adhesion-promoting additives differing from constituent (a).

11. The blend according to claim 10, wherein constituent (b-1) consists of or comprises
(b-1a) one or more (meth)acrylate monomers, preferably selected from the group consisting of 2-hydroxyethyl methacrylate (HEMA), bisphenol A glycidyl methacrylate (bis-GMA), 7,7,9-trimethyl-4,13-dioxo-5,12-diazahexadecane-1,16-dioxy dimethacrylate (UDMA), triethylene glycol dimethacrylate (TEDMA), tetraethylene glycol dimethacrylate,, polyethylene glycol dimethacrylate, trimethylolpropane trimethacrylate (TMPTMA), dodecanediol dimethacrylate (DODMA), glycerol di(meth)acrylate, 1,fi-hexanediol dimethacrylate (HEDMA), ethoxylated bisphenol A dimethacrylate, pentaerythritol dimethacrylate, pentaerythritol tri(meth)acrylate and dipentaerythritol penta(meth)acrylate,
and/or
(b-1b) one or more monomers selected from the group consisting of compounds of the structure Q^{a}(MXₑ)ₕ, wherein the following applies:
- Q^{a} is a polyalicyclic structure element and has - independently of the meaning of the structure element Q in compounds of constituent (a) - the meaning given above for Q,
- h is a natural number selected from the group of natural numbers 1, 2, 3 and 4,
- each X represents a structure element which - independently of any further structure elements X - is selected from the group consisting of
-O-(C=O)-CH=CH₂, -O-(C=O)-C(CH₃)=CH₂,
-(C=O)-CH=CH₂, -(C=O)-C(CH₃)=CH₂ and -O-CH=CH₂,
- each index e is a natural number which, independently of any further indices e, is selected from the group of natural numbers 1, 2, 3 and 4,
- each M represents a structure element which - independently of any further structure elements M - binds the polyalicyclic structure element Q^{a} with e structure elements X in the structure Q^{a}(MXₑ)ₕ.

12. A product obtainable by curing a compound according to one of claims 1 to 7, a mixture according to claim 9 or a blend according to one of claims 10 and 11.

13. The compound according to one of claims 1 to 7, the mixture according to claim 9, the blend according to one of claims 10 and 11 or the product according to claim 12 as a dental material or for use as a dental material.

14. Use of a compound according to one of claims 1 to 7, a mixture according to claim 9. a blend according to one of claims 10 and 11 or a product according to claim 12 for the preparation of a dental material.

15. A method for preparing a product, preferably a dental product, with the following steps:
(i) providing a compound according to one of claims 1 to 7, a mixture according to claim 9 or a blend according to one of claims 10 and 11 as a first component,
(ii) optionally preparing a preparation by mixing the first component with one or more further components, preferably with one or more further dental materials,
(iii) curing the constituent(s) from step (i) or the preparation according to step (ii), the curing preferably taking place chemically and/or being light induced or thermally induced.

## Revendications

1. Composé de structure (HO)_{w}-P(=O)-[G-(L)ₓ-Q(YZ)_{b}]_{y},
dans lequel, ici et dans ce qui suit :
- chaque Q représente, indépendamment des autres groupes Q éventuels, un élément structurel polyalicyclique saturé ou oléfiniquement insaturé choisi dans le groupe comprenant les radicaux hydrocarbure bicycliques, tricycliques, tétracycliques, pentacycliques et hexacycliques, Q ne portant pas d'autres substituants, ou un ou plusieurs substituants choisis dans le groupe comprenant des groupes alkyle, des groupes alcoxy, des halogénoatomes et des groupes trifluorométhyle,
- l'indice b est un nombre naturel choisi dans le groupe des nombres naturels 1, 2 et 3,
- chaque Z désigne un élément structurel qui est choisi indépendamment des autres éléments structurels éventuels dans le groupe comprenant
- (C=O)-CH=CH₂, - (C=O)-C(CH₃)=CH₂ et -CH=CH₂,
- chaque Y désigne un élément structurel qui contient, indépendamment d'autres éléments structurels Y éventuels, un ou plusieurs atome(s) de N et l'élément structurel polyalicyclique Q est lié à Z de telle sorte que dans la chaîne d'atomes reliant Q à Z, un ou plusieurs atomes de N soient contenus,
- chaque élément structurel G désigne, indépendamment d'autres éléments structurels G éventuels, soit 0, soit NH,
- chaque L désigne un élément structurel qui, indépendamment d'autres éléments structurels éventuels L, relie le groupe G à l'élément structurel polyalicyclique Q,
- l'indice w est choisi dans le groupe des nombres naturels 0, 1 et 2,
- chaque indice x désigne, indépendamment d'autres indices x éventuels, soit 0, soit 1,
- l'indice y est choisi dans le groupe des nombres naturels 1, 2 et 3,
le total de w + y = 3.

2. Composé selon la revendication 1, le composé présentant la structure suivante
(HO)_{w}-P(=O)-[G-(CH₂)ₖ-(A-C(=O))ᵣ(E)ⱼ-(CH₂)ₙ-Q(YZ)_{b}]_{y},
dans laquelle :
- chaque indice k est un nombre naturel qui est choisi, indépendamment d'autres indices k éventuels, dans le groupe des nombres naturels 0 à 12, de préférence dans le groupe des nombres naturel 0 à 8,
- chaque élément structurel A désigne, indépendamment d'autres éléments structurels A éventuels, soit O, soit NH,
- chaque élément structurel E désigne, indépendamment d'autres éléments structurels E éventuels, soit O, soit NH,
- chaque indice r désigne, indépendamment d'autres indices r éventuels, soit 0, soit 1,
- chaque indice j désigne, indépendamment d'autres indices j éventuels, soit 0, soit 1,
- chaque indice n désigne, indépendamment d'autres indices n éventuels, soit 0, soit 1,
Où, si k = 0, cela signifie que l'indice j = 0 ou l'indice r = 0.

3. Composé selon la revendication 1 ou 2, comprenant un ou deux groupes fonctionnels ou plus, choisis dans le groupe comprenant un ester, un uréthane, un N-acyluréthane, une urée, une N-acylurée et un amide, la fonction amide n'étant pas reliée directement à un atome de N, un atome de 0 ou un groupe carbonyle.

4. Composé selon l'une des revendications précédentes, **caractérisé en ce que** le groupement YZ comprend un groupe fonctionnel choisi dans le groupe comprenant un ester, un uréthane, un N-acyluréthane, une urée, une N-acylurée et un amide, la fonction amide n'étant pas reliée directement à un atome de N, un atome de O ou un groupe carbonyle.

5. Composé selon l'une des revendications précédentes, **caractérisé en ce que** le groupement YZ comprend un groupe fonctionnel choisi dans le groupe comprenant un uréthane, un N-acyluréthane, une urée, une N-acylurée et un méthacrylamide, au moins l'un des atomes de N de ce groupe fonctionnel se trouvant dans la chaîne d'atomes reliant Q à Z.

6. Composé selon l'une des revendications précédentes, **caractérisé en ce que** le groupement YZ présente une structure choisie dans le groupe comprenant
(-CH₂)ₙ-O-C(=O)-NH-Z,
(-CH₂)ₙ-O-C(=O)-NH-(CH₂)ₘ-O-Z,
(-CH₂)ₙ-NH-C(=O)-NH-Z,
(-CH₂)ₙ-NH-C(=O)-NH-(CH₂)ₘ-O-Z,
(-CH₂)ₙ-NH-Z,
et
(-CH₂)ₙ-NH-C(=O)-O-(CH₂)ₘ-O-Z,
où Z et n ont la signification indiquée ci-dessus et où :
- chaque indice m est un nombre naturel qui est choisi indépendamment d'autres indices m éventuels, dans le groupe des nombres naturels 1 à 12, de préférence dans le groupe des nombres naturels 2 à 8.

7. Composé selon l'une des revendications précédentes, dans lequel l'élément structurel Q désigne un radical tricyclo[5.2.1.0^{2,6}]décane, un radical tricyclo[5.2.1.0^{2,6}]déc-3-ène, un radical tricyclo [3.3.1.1^{3,7}]décane ou un radical bicyclo [2.2.1]heptane.

8. Procédé de production d'un composé de structure (HO)_{w}-P(=O)-[G-(L)ₓ-Q(YZ)_{b}]_{y}, selon l'une des revendications précédentes, ou d'un mélange comprenant au moins un composé de structure (HO)_{w}-P(=O)-[G-(L)ₓ-Q(YZ)_{b}]_{y}, selon l'une des revendications précédentes, comportant les étapes suivantes :
(i) la mise à disposition d'un composé HG-(L)ₓ-Q(YZ)_{b},
(ii) la réaction du composé de l'étape (i) avec du POCl₃ ou un oxyde de phosphore (V), de préférence en présence d'une amine, de manière préférée d'une amine tertiaire,
(iii) l'hydrolyse du produit réactionnel formé à l'étape (ii),
où G, L, Q, Y, Z, w, x, b et y ont respectivement la signification mentionnée ci-dessus, et
où la quantité molaire de composé de l'étape (i) par équivalent d'atome de phosphore se situe dans une plage de 0,5 à 6, de préférence dans une plage de 0,6 à 5, de manière préférée dans une plage de 0,7 à 4, de manière davantage préférée dans une plage de 0,8 à 3,5.

9. Mélange comprenant un ou deux composés différents ou plus selon l'une des revendications 1 à 7, de préférence pouvant être produit d'après un procédé selon la revendication 8.

10. Mélange durcissable comprenant
(a) un ou plusieurs composés selon les revendications 1 à 7 ou un mélange selon la revendication 9,
et
(b) un ou plusieurs autres composants choisis dans le groupe comprenant
(b-1) des monomères différents du composant (a) qui sont copolymérisables avec le composant (a), de préférence des monomères photopolymérisables,
(b-2) une ou plusieurs substances de charge, de préférence une ou plusieurs substances de charge à une nano-échelle,
(b-3) des photo-initiateurs et des initiateurs pour le durcissement chimique,
(b-4) des inhibiteurs de polymérisation,
(b-5) des solvants,
et
(b-6) des additifs favorisant l'adhérence différents du composant (a).

11. Mélange selon la revendication 10, dans lequel le composant (b-1) consiste en ou comprend
(b-1a) un ou plusieurs monomères de (méth)acrylate, choisis de préférence dans le groupe comprenant le 2-hydroxyéthylméthacrylate (HEMA), le bisphénol-A-glycidyl-méthacrylate (Bis-GMA), le 7,7,9-triméthyl-4,13-dioxo-5,12-diazahexadécane-1,16-dioxy-diméthacrylate (UDMA), le triéthylène glycol-diméthacrylate (TEDMA), le tétraéthylèneglycol-diméthacrylate, le polyéthylèneglycol-diméthacrylate, le triméthylolpropane-triméthacrylate (TMPTMA), le dodécanediol-diméthacrylate (DODMA), le glycérine-di(méth)acrylate, le 1,6-hexanedioldiméthacrylate (HEDMA), le bisphénol-A-diméthacrylate éthoxylé, le penta-érythritoldiméthacrylate, le penta-érythritol-tri(méth)acrylate et le dipenta-érythritol-penta(méth)acrylate,
et/ou
(b-1b) un ou plusieurs monomères choisis dans le groupe comprenant les composés de structure Q^{a}(MXₑ)ₕ, où
- Q^{a} est un élément structurel polyalicyclique et a, indépendamment de la signification de l'élément structurel Q dans les composés du composant (a), la signification indiquée ci-dessus pour Q,
- h est un nombre naturel choisi dans le groupe des nombres naturels 1, 2, 3 et 4,
- chaque X désigne un élément structurel qui, indépendamment d'autres éléments structurels X éventuels, est choisi dans le groupe comprenant
-O-(C=O)-CH=CH₂, -O-(C=O)-C(CH₃)=CH₂,
-(C=O)-CH=CH₂, -(C=O)-C(CH₃)=CH₂, et -O-CH=CH₂,
- chaque indice e est un nombre naturel qui, indépendamment d'autres indices e éventuels, est choisi dans le groupe des nombres naturels 1, 2, 3 et 4,
- chaque M désigne un élément structurel qui, indépendamment d'autres éléments structurels M éventuels, dans la structure Q^{a}(MXₑ)ₕ, relie l'élément structurel polyalicyclique Q^{a} à e éléments structurels.

12. Produit pouvant être obtenu par durcissement d'un composé selon l'une des revendications 1 à 7, d'un mélange selon la revendication 9 ou d'un mélange selon l'une des revendications 10 et 11.

13. Composé selon l'une des revendications 1 à 7, mélange selon la revendication 9, mélange selon l'une des revendications 10 et 11 ou produit selon la revendication 12, en tant que matériau dentaire ou pour l'utilisation en tant que matériau dentaire.

14. Utilisation d'un composé selon l'une des revendications 1 à 7, d'un mélange selon la revendication 9, d'un mélange selon l'une des revendications 10 et 11 ou d'un produit selon la revendication 12, pour la production d'un matériau dentaire.

15. Procédé de production d'un produit, de préférence d'un produit dentaire, comportant les étapes suivantes :
(i) la mise à disposition d'un composé selon l'une des revendications 1 à 7, d'un mélange selon la revendication 9 ou d'un mélange selon l'une des revendications 10 et 11 comme premier composant,
(ii) éventuellement, la production d'une préparation par mélange du premier composant mis à disposition avec un ou plusieurs autres composants, de préférence avec un ou plusieurs autres matériaux dentaires,
(iii) le durcissement du ou des composant(s) de l'étape (i) ou de la préparation selon l'étape (ii), le durcissement étant induit chimiquement et/ou par la lumière et/ou thermiquement.
